# EUROPEAN PATENT SPECIFICATION

(11) **EP 1 322 327 B1**
(45) Date of publication and mention of the grant of the patent: **04.06.2008**
(21) Application number: 01972273.5
(22) Date of filing: 02.10.2001
(51) Int. Cl.: A61K 39/02, C12N 15/63, C12N 15/68, C12N 1/21

(54) **STABILISATION OF PLASMID INHERITANCE IN BACTERIA BY PREVENTING MULTIMERISATION**
STABILISIERUNG DER PLASMIDVERERBUNG IN BAKTERIEN DURCH VERHINDERUNG DER MULTIMERBILDUNG
STABILISATION DE L'HEREDITE PASMIDIQUE CHEZ DES BACTERIES PAR UNE PREVENTION DE FORMATION DE MULTIMERES

(30) Priority: 03.10.2000 GB 0024203
(43) Date of publication of application: 02.07.2003
(73) Proprietor: Acambis Research Limited, Cambridge CB1 9PT (GB)
(72) Inventor: TURNER, Arthur, K., c/o Acambis Research Limited, Cambridge CB1 9PT (GB); STEPHENS, Jonathan,C, c/o Acambis Research Limited, Cambridge CB1 9PT (GB)
(74) Representative: Campbell, Patrick John Henry
(86) International application number: PCT/GB2001/004382
(87) International publication number: WO 2002/028423

(56) References cited:
- WO-A-00/32047
- KRAUSE M ET AL: "IDENTIFICATION OF A MULTIMER RESOLUTION SYSTEM INVOLVED IN STABILIZATION OF THE SALMONELLA-DUBLIN VIRULENCE PLASMID PSDL2" JOURNAL OF BACTERIOLOGY, vol. 173, no. 18, 1991, pages 5754-5762, XP001056657 ISSN: 0021-9193
- PULLINGER G D ET AL: "A SALMONELLA-DUBLIN VIRULENCE PLASMID LOCUS THAT AFFECTS BACTERIAL GROWTH UNDER NUTRIENT-LIMITED CONDITIONS" MOLECULAR MICROBIOLOGY, vol. 6, no. 12, 1992, pages 1631-1643, XP001056660 ISSN: 0950-382X
- EASTER CARLA L ET AL: "Contribution of different segments of the par region to stable maintenance of the broad-host-range plasmid RK2." JOURNAL OF BACTERIOLOGY, vol. 179, no. 20, 1997, pages 6472-6479, XP002191826 ISSN: 0021-9193

## Description

The invention relates to the stabilisation of plasmid inheritance in bacteria for use in vaccines.

### Background to the invention

The principle behind vaccination is to induce an immune response in the host, thus providing protection against subsequent challenge with a pathogen. This may be achieved by inoculation with a live attenuated strain of the pathogen, i.e. a strain having reduced virulence such that it does not cause the disease caused by the virulent pathogen.

The advent of recombinant DNA technology has greatly speeded up the development of vaccines. In particular, this technology has led to the development of vaccines which use bacteria transformed with an expression vector encoding a heterologous antigen from a pathogen. This means that a well characterised bacterial strain can be used to express a wide variety of antigens and to act as a carrier to present antigens to the immune system. Live attenuated pathogens are commonly used as the carrier bacteria in such vaccines

Ideally, the bacteria used in vaccines are genetically defined, attenuated, well-tolerated by the recipient animal or human, and retain immunogenicity. Using modern genetic techniques, it is now possible to construct genetically defined attenuated bacterial strains in which stable attenuating deletions have been created. A number of site directed mutants of *Salmonella* have been created using this type of technology. Mutations in a large number of genes have been reported to be attenuating, including the aro genes (e.g. *aroA, aroC, aroD* and *aroE*), *pur, htrA, ompR, ompF, ompC, galE, cya, crp* and *phoP.*

A major problem with using recombinant plasmids to direct expression of heterologous antigens in vaccines is their instability. Most recombinant plasmids are inherited relatively stably in laboratory adapted strains of *E. coli* or other bacteria, but are often rapidly lost from actively growing populations of other bacterial species, for example *Salmonella typhi.*

The loss of expression plasmids from a live vaccine organism after administration to a host has a deleterious effect on the immune response generated. Improvement of the stability of such expression plasmids is therefore a key requirement in the successful development of a live vaccine. A number of systems for achieving plasmid maintenance in live vaccines have been suggested:
1. Maintenance of plasmids is frequently achieved *in vitro* by continuous selection for a plasmid encoded marker such as antibiotic resistance. However, this is not an effective means of achieving plasmid stability *in vivo,* where concentrations of antibiotics cannot be maintained.
2. The genetic material encoding the desired heterologous antigen may be incorporated into the bacterial chromosome dispensing with the need for a plasmid vector altogether. However, the level of antigen expression from a single chromosomal copy is generally insufficient to generate a functional immune response.
3. Bacterial strains lacking an essential gene are used in conjunction with a plasmid carrying the essential gene missing from the bacterial chromosome. Any bacterial cells which lose the plasmid die. The best example of this is the asd "balanced lethal" system (Curtis et al. Immunol. Invest., 1989 18(1-4): 583-596). In the *asd* balanced lethal system, the *asd* mutation in the chromosome makes the cells prone to lysis if grown in the absence of diaminopimelic acid. The *asd* gene is carried by the plasmid, so that loss of the plasmid results in lysis of the cells. This system does not improve plasmid inheritance; it merely removes from the population those cells that have lost the plasmid. Thus if the plasmid shows poor inheritance, the bacteria will show poor growth and this is likely to increase significantly the attenuation of the *Salmonella typhi* strain leading to reduced activity as a vaccine. Delivery of heterologous antigens using this system worked well in *Salmonella typhimurium,* where plasmids are inherited relatively stably, but gave poor results in *Salmonella typhi* where plasmids are inherited less efficiently (Tacket et al, Infect. Immun., 1997. 65 (8): 3381-3385). Balanced lethal systems are therefore satisfactory for *in vitro* maintenance of plasmids but are not effective *in vivo.*
4. Galen et al (Infect. Immun., 1999, 67(12): 6424-6433) used a combination of a stabilising locus (*par* from plasmid PSC101), a partition system (*parA* from plasmid R1) and the *hok-sok* suicide system from plasmid R1, in order to enhance plasmid maintenance. None of the systems used in Galen *et al* incorporated a site-specific recombination system.

Even with incorporation of systems (1), (3) or (4) onto a recombinant plasmid, unstable inheritance continues to be a problem after prolonged replication (more than one culture passage) in bacteria such as *Salmonella typhi.* Of systems (1) to (4) only system (4), and specifically the *par* and *parA* sequences employed in (4), function to enhance the inheritance of the plasmid; in systems (1) to (3) the plasmid continues to be lost from the bacterial population, but is nevertheless maintained through the removal of cells lacking the plasmid by killing mechanisms. Use of such killing mechanisms with unstable plasmids leads to additional attenuation in the already attenuated vaccine strains, rendering them less effective.

The reasons for unstable inheritance of recombinant plasmids are probably several fold. Summers et al (Mol. Microbiol.,1993. 8(6):1031-8) have suggested that one possible factor in plasmid instability is the formation of plasmid multimers which arise in bacterial cells as a result of homologous recombination.

Plasmids occur naturally in most bacteria. The majority of these are of low copy number but their inheritance can be very stable, even in the absence of selection. Several genetic systems that contribute to the stability of naturally occurring systems have been described. These.include:
- partition functions which increase the probability that dividing cells each receive at least one copy of the plasmid;
- transfer functions, enabling the spread of the plasmid to plasmid-free hosts by conjugation (Easter et al, J. Bacterol., 1997.79(20): 6472-64.79);
- suicide mechanisms which are activated upon loss of the plasmid from the cell (Pecote et al, Appl. Environ. Microbial., 1997. 63(5): 1917-1924); and
- multimer resolution systems which involve site-specific recombinases.

Multimer resolution systems confer stability by resolving plasmid multimers into single plasmid copies and hence decreasing the chance of plasmid free daughter cells being generated during random segregation at cell division. A number of site-specific recombination systems which act to resolve plasmid multimers into monomers have been identified.

### Summary of the invention

As part of a programme to develop new strains of live attenuated bacteria for use in vaccines, we developed an expression plasmid to express heterologous polypeptides, typically heterologous antigens from pathogens, in these bacteria. The plasmid was found to be stably inherited in live attenuated bacteria when antibiotic selection was applied, but lost from the same strains when grown in the absence of antibiotic selection. We modified the plasmid to attempt to increase its stability.

We introduced a cassette containing a DNA fragment encoding a site-specific recombinase and the recognition element for the recombinase into the plasmid. The modified plasmid was found to be significantly more stable when expressed in live attenuated bacteria grown in the absence of antibiotic selection than the parental plasmid it was derived from. The plasmid containing the cassette was also found to be more stable than the parental plasmid when both were expressed in attenuated bacteria and antibiotic selection applied.

In the absence of antibiotic selection the plasmid containing the cassette was still present in 100% of the bacterial cells in a culture after 40 to 45 generations whereas the parental plasmid lacking the cassette was present in less than 10% of the cells after the same number of generations. In similar experiments where antibiotic selection was applied the modified plasmid was also more stable than the parental plasmid; typically, 100% of cells still contained the plasmid with the cassette whereas only 80% of cells contained the parental plasmid lacking the cassette.

Accordingly, the present invention provides a vaccine comprising a pharmaceutically acceptable carrier or diluent and a bacterium, wherein the bacterium contains:
(i) a DNA sequence encoding a site-specific recombinase; and
(ii) a plasmid comprising a recognition element for the recombinase and a DNA sequence encoding a heterologous polypeptide.

Further improvements were made to the cassette by removing the intervening sequence between the sequence encoding the site-specific recombinase and the recognition element from the cassette, so that the two functional loci were approximately adjacent to each other on a 1.5kb fragment. Removing the unnecessary sequences from the cassette reduced its size, making it and plasmids containing it smaller and hence more amenable to manipulation. The smaller version of the cassette functions similarly to its longer parent sequence to stabilise plasmid inheritance.

The plasmid containing the cassette was also modified by introducing a transcription termination sequence between the recognition element and the promoter of the gene encoding the heterologous polypeptide. This was done because a high level of constitutive expression of the heterologous polypeptide from the plasmid containing the cassette was observed, presumably from an active promoter within the stabilising cassette. The transcription termination sequence ensured that the expression of the heterologous polypeptide was controlled from the intended promoter only. It may also be possible to delete the region of the cassette responsible for the promoter activity whilst retaining the stabilisation activity.

### Detailed description of the invention

### Plasmids useful in the invention

The plasmids for use in the invention comprise a recognition element for a site-specific recombinase and a DNA sequence encoding a heterologous polypeptide. Preferably the plasmid also includes the DNA sequence encoding the site-specific recombinase. Alternatively, the DNA sequence encoding the recombinase may be present either on a separate plasmid or integrated into the bacterial chromosome. The nature of the recombinases and recognition elements that may be used in the invention is discussed in detail below.

When the plasmid comprises both the recognition element and the DNA sequence encoding the recombinase the length of the intervening sequence between the two is preferably minimal. Reducing the size of the cassette by decreasing the size of, or removing, the intervening sequence means that the cassette, and plasmids containing it, are smaller and hence more easily manipulatable using recombinant DNA techniques. Typically, the recognition element and the sequence encoding the recombinase are less than 1.5kb, preferably less than 0.5kb and more preferably less than 0.2kb apart.

In one embodiment of the invention there is a transcriptional termination sequence between the recognition element and the promoter of the gene encoding the heterologous polypeptide. The transcriptional termination sequence ensures that expression of the heterologous polypeptide is from the intended promoter only. Any suitable transcriptional termination sequence may be used, preferably a strong transcriptional termination sequence which allows minimal or no transcription. In a preferred embodiment of the invention, the 5S *rrnB* transcription terminators, *rrnB* T1 and *rrnB* T2 are used. The nucleotide sequences of these terminators are shown in SEQ ID NO: 1.

Preferably, the expression cassette for the heterologous polypeptide and/or recombinase is composed of a promoter a transcription initiation site, a ribosome binding site (RBS), the coding sequences for the heterologous polypeptide or recombinase, a translational stop codon and a transcription terminator.

The expression of the heterologous polypeptide may be driven by a variety of promoters. Preferably a prokaryotic promoter, and in particular a promoter suitable for the chosen strain of bacterium, will be used. In particular, expression may be driven by the *nirB* promoter or the *htrA* promoter (see USP 5,683,700 and WO 95/20665, respectively). The *pagC* or *ssaH* promoters may also be used. Typically expression of the recombinase will be driven by its own endogenous promoter.

The plasmid may also comprise a selection marker such as an antibiotic resistance gene. Any suitable selection marker may be used. Typically an ampicillin, tetracyclin, chloramphenical or kanamycin resistance gene is used. In a preferred embodiment of the invention, a kanamycin resistance marker is used. The plasmid employed in the invention may be a plasmid known in the art modified to include the recognition element and optionally also the sequence encoding the recombinase.

The plasmid of the present invention may also include one or more other systems for increasing plasmid stability or plasmid maintenance. Suitable systems include a suicide system, a balanced lethal system, a partition system and the *par* locus from the plasmid pSC101 (Galen *et al,* supra).

In the case of the balanced lethal system, a gene essential for bacterial survival is deleted from the bacterial chromosome. The gene deleted from the chromosome is present on the plasmid; loss of the plasmid will therefore mean the bacterium is unable to survive. In one embodiment of the invention the balanced lethal system used is the *asd* system described in (3) above.

In the case of the suicide system, the plasmid or bacterial chromosome encodes a potent cell-killing agent that destroys plasmid free segregants, while bacteria which retain the plasmid are protected by a plasmid encoded antidote to the cell-killing agent. Typically, the cell-killing agent and/or the RNA encoding it are more stable than the antidote or the RNA encoding the antidote. This means after plasmid loss the cell-killing agent will be present in the cell for longer or at a higher concentration than the antidote and hence loss of the plasmid will result in the death of the bacterium. Generally, the cell-killing agent is a toxin and may be a membrane pore or lysogen. The antidote may be an antisense RNA capable of blocking expression of the gene encoding the cell-killing agent, an enzyme responsible for the breakdown of the agent or a protein which binds to the agent and inactivates it. In one embodiment of the invention, the suicide system is the *hok-sok* system (Galen *et al* supra).

In the case of partition systems, genetic elements are included in the plasmid which increase the probability at cell division that each daughter cell receives a copy of the plasmid. Such systems typically act by an active physical separation of plasmid molecules into daughter cells. Suitable partition systems for use in the invention include the *par A* centromere-like active partitioning system from the plasmid pR1 (Galen *et al* supra).

### Recombinases and recognition elements

Any site-specific recombinase which stabilises inheritance of the plasmid containing the recognition element may be used in the invention. The recombinase is generally a resolvase. Resolvases are able to resolve plasmid multimers. The site-specific recombinase may also be an integrase or an invertase, some of which have resolvase activity.

Examples of resolvases which may be used in the invention include the Cre recombinase of plasmid P1, the *E. coli* XerC (ArgR) protein, the D protein recombinase of plasmid F, the ParA recombinases of plasmids RP4 and RK2, the site-specific recombinase of plasmid R1 resolvases encoded by the Tn3-like transposable genetic elements and the Rsd resolvase from the *Salmonella dublin* virulence plasmid. Other resolvases not mentioned here but which have a similar activity may also be used in the invention. In a particularly preferred embodiment of the invention, the recombinase is the Rsd resolvase. The Rsd resolvase may be encoded by
(a) a DNA molecule comprising the nucleotide sequence from positions 680 to 1459 of SEQ ID NO: 1,
(b) a DNA molecule which hybridises to the complement of (a) or
(c) a DNA molecule which encodes the same amino acid sequence as the DNA molecule of (a) or (b) but which is a degenerate form of the DNA molecule of (a) or (b).

In addition to the sequences encoding the resolvase the bacterium may also comprise a nucleotide sequence which encodes a polypeptide necessary for, or which facilitates, multimer resolution by the site-specific recombinase employed in the invention. These sequences may be present on the plasmid of the invention, a separate plasmid or on the bacterial chromosome.

Recognition elements which may be used in the present invention include those for the above recombinases. Any recognition element recognised by the site-specific recombinase employed may be used. Suitable recognition elements include those sites recognised by the XerC site-specific recombinase, such as the *cer* site of plasmid ColE1 and the similar *ckr* site of plasmid CoIK (Summers et al, Mol. Genet. Genes., 201(2): 334 - 338), the *psi* site of plasmid pSC101 and the *cer* like site of plasmid pHS-2 from *Shigella flexneri.* Other recognition elements which may be used include the *crs* site from the *Salmonella dublin* virulence plasmid, the *loxP* site of plasmid P1; the *rfs* site of the F plasmid and the *res* site of the Tn3-like transposable genetic element. Elements not mentioned specifically here but which have similar activity may also be used. In a preferred embodiment of the invention the *crs* recognition element is employed as the recognition element. The *crs* recognition element may have the sequence of
(a) nucleotides 54 to 556 of SEQ ID NO:1 or the complement thereof, or
(b) a DNA molecule which hybridises to a DNA molecule comprising the sequence of nucleotides 53 to 556 of SEQ ID NO:1 or to the complement thereof.

The particular recombinase employed may be adapted for use in the bacterium strain employed. For example, mutation screens may be used to isolate variants of known recombinases which act particularly well in the chosen strain of bacterium in a combination with the above-mentioned assays.

Two or more different site-specific recombinases may be used at the same time to stabilise two or more different plasmids in the same bacterial cell. In such embodiments, each type of plasmid to be stabilised will typically contain a different recognition element specific to one of the recombinases used. Alternatively, a single site-specific recombinase may be used to stabilise two or more different plasmids in the same cell, with each plasmid containing a recognition element for the recombinase.

A homologue of the polynucleotide sequence encoding the Rsd resolvase from positions 680 to 1459 of SEQ ID NO: 1 and/or a homologue of the nucleotide sequence of the *crs* recognition element from positions 54 to 556 of SEQ ID NO: 1 may be used in the invention. Typically, a homologue has at least 70% sequence identity to the corresponding sequence in SEQ ID NO: 1, preferably at least 80 or 90% and more preferably at least 95%, 97% or 99% sequence identity. Such sequence identity may exist over a region of at least 15, preferably at least 30, for instance at least 40, 60 or 100 or more contiguous nucleotides.

Methods of measuring polynucleotide homology are well known in the art. For example, the UWGCG Package providing the BESTFIT program can be used to calculate homology, e.g. on its default settings (Devereux et al (1984) Nucleic Acids Research 12, p387-395). The PILEUP and BLAST algorithms can also be used to calculate homology or line up sequences (typically on their default settings), for example as described in Altschul (1993) J Mol Evol 36: 290-300 or Altschul et al (1990) J Mol Biol 215: 403-10.

The homologues typically hybridise with the corresponding sequence in SEQ ID NO: I at a level significantly above background. The signal level generated by the interaction between the homologue and the sequence of SEQ ID NO: 1 is typically at least 10 fold, preferably at least 100 fold, as intense as background hybridisation. The intensity of interaction may be measured, for example, by radiolabelling the probe, e.g. with 32P. Selective hybridisation is typically achieved using conditions of medium to high stringency, for example 0.03M sodium chloride and 0.003M sodium citrate at from about 50°C to about 60°C.

The homologue may differ from corresponding sequence in SEQ ID NO: 1 by at least 1, 2, 5, 10 or more substitutions, deletions or insertions over a region of at least 30, for instance at least 40, 60 or 100 or more contiguous nucleotides, of the homologue. Thus, the homologue may differ from the corresponding sequence in SEQ ID NO: I by at least 1, 2, 5, 10,30 or more substitutions, deletions or insertions.

A homologue Rsd resolvase or *crs* recognition element may be tested for its ability to stabilise a test plasmid. In the case of testing a potential recognition element, the candidate element may be inserted into a test plasmid and the plasmid's stability assessed in one or more bacterial strains in the presence or absence of the recombinase and/or in comparison to the plasmid lacking the element. A sequence encoding a homologue recombinase may be assessed for its ability to stabilise a plasmid containing the recognition element for that recombinase by comparing plasmid stability in the presence or absence of the recombinase.

Plasmid stability may be assessed using the plasmid inheritance assay described in the Examples below. In general terms, this assay involves providing a colony or culture of bacteria containing the plasmid. The bacteria are used to inoculate culture medium. The culture is grown for a period of time and may be passaged. The proportion of bacteria which still contain the plasmid at the end of the culture or at a series of time points during the culture is determined, for example by determining the proportion of the bacteria which contain a selection marker present on the plasmid (e.g. an antibiotic resistance marker).

Plasmid stability can also be assessed *in vivo* by vaccinating mice with bacteria such as *S. typhimurium* containing the plasmid. The proportion of bacteria in spleens which contain the plasmid can then be determined.

Furthermore, plasmid stability may be assessed by isolating the plasmid from a bacterium transformed with the plasmid and determining the proportion of plasmid in monomeric and multimeric forms; the presence of multimeric forms of the plasmid indicates that plasmid monomers are not being resolved and that the plasmid may thus be unstable. Typically, plasmid DNA is isolated after a predetermined time, number of bacterial divisions or passages, and isolation is preferably done at a series of time points to assess plasmid stabilisation over time.

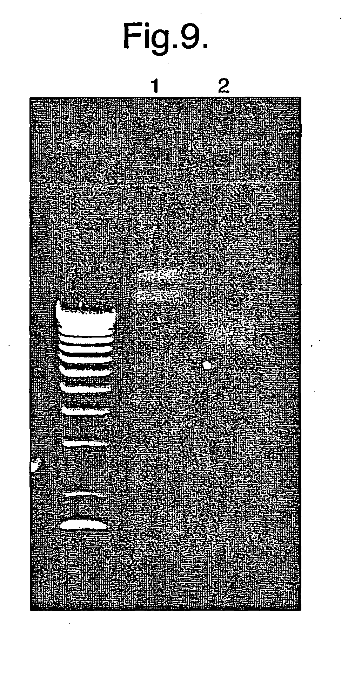

The proportion of plasmid DNA in momeric and multimeric forms may be determined by electrophoresing the plasmid DNA on an agarose gel and visualising the different momeric and multimeric forms of the plasmid, typically using ethidium bromide staining and U.V. illumination. Multimeric forms of the plasmid should move more slowly through the gel than monomeric forms hence allowing the two to be distinguished. Typically the plasmid DNA is run uncut and so it is in its supercoiled form and hence resolution of the different forms of plasmid will be easier. In some cases a ladder of different size multimers will be seen on the gel and the relative proportions of each form can be assessed. A typical gel is shown in Figure 9, in which lane 2 contains a plasmid according to the invention and lane 1 contains a smaller control plasmid.

Recognition elements and/or recombinases for use in the invention will preferably stabilise, or increase the stability of, test plasmids in these assays. Typically they will reduce or eliminate multimeric forms of the test plasmid in these assays.

### DNA vaccines

In one embodiment of the invention, the vaccine is a DNA vaccine. Preferably, in such embodiments the carrier bacterium in the vaccine is an invasive bacterium and expression of the heterologous antigen is driven by a eukaryotic promoter. In such embodiments of the invention, expression of the heterologous antigen will typically occur inside the cells of the vaccinated individual.

It has been shown that invasive bacteria (bacteria which can enter host cells) can be used to deliver DNA vaccines. US Patent Number 5, 877,159 provides a description of the principle of DNA vaccines and how such vaccines may be provided. The methods and vaccines employed in USP 5, 877,159 may be applied in combination with the present invention.

Invasive bacteria include bacteria that are naturally capable of entering the cytoplasm or nucleus of animal cells, as well as bacteria which are not naturally invasive but which have been genetically engineered to enter the cytoplasm or nucleus of animal cells. It has been shown also that *Salmonella* strains can be used to deliver DNA vaccines (Darji et al, 1997, Cell, 91, 765 to 775; Paglia et al, Blood, 1998, 92 (9) 3172 to 3176).

The promoter used to express the heterologous antigen in this embodiment is functional in eukaryotic cells. The promoter may be a viral promoter, such as the SV40, CMV or RSV promoter or a eukaryotic cell promoter, such as the β-casein, uteroglobin, β-actin or tyrosinase promoter. Preferably the promoter used to drive expression of the heterologous antigen will be one capable of being expressed in an antigen presenting cell (APC).

### Bacteria useful in the invention

The bacteria that are used to make the vaccines of the invention are generally those that infect by the oral route. The bacteria may be those that invade and grow within eukaryotic cells and/or colonise mucosal surfaces. The bacteria are generally Gram-negative but in some embodiments involving intra-nasal immunisation gram positive bacteria may be used. The bacteria are generally attenuated pathogens.

The bacteria used may be from the genus *Escherichia, Salmonella, Shigella, Vibrio, Haemophilus, Neisseria, Yersinia, Bordetella, Brucella, Listeria, Rickettsia, Klebsiella, Aeromonas, Franciesella, Corynebacterium, Citrobacter, Chlamydia, Mycobacterium. Legionella, Rhodococcus, Pseudomonas, Helicobacter, Bacillius, Leishmania* or *Erysipelothrix.*

Examples of particularly preferred bacteria for use in the invention are *Escherichia coli -* which can be a cause of diarrhoea in humans; *Salmonella typhimurium* - the cause of salmonellosis in several animal species; *Salmonella typhi -* the cause of human typhoid; *Salmonella enteritidis -* a cause of food poisoning in humans; *Salmonella choleraesuis -* a cause of salmonellosis in pigs; *Salmonella dublin -* a cause of both a systemic and diarrhoel disease in cattle, especially of new-born calves; *Haemophilus influenza* - a cause of meningitis; *Neisseria gonorrhoeae -* a cause of gonorrhoeae; *Yersinia enterocolitica* - the cause of a spectrum of diseases in humans ranging from gastroenteritis to fatal septicemic disease; *Bordetella pertussis -* the cause of whooping cough; and *Brucella abortus* - a cause of abortion and infertility in cattle and a condition known as undulant fever in humans.

Strains of *E. coli* and *Salmonella* are particularly useful in the invention. *Salmonella* are potent immunogens and are able to stimulate systemic and local cellular and antibody responses. In particular an attenuated strain of *Salmonella typhi* is preferred for use in the invention. Preferred *Salmonella typhi* strains for use in the present invention include CVD908-htrA (ΔaroC ΔaroD ΔhtrA) and CVD908 (ΔaroC ΔaroD) (Tacket et.al, 1997, Infection & Immunity, 65 (2),452 to 456). Attenuated strains of enterotoxigenic *E.coli* ("ETEC") may also be used. ETEC is a class of *E. coli* that cause diarrhoea. ETEC colonise the proximal small intestine. A standard ETEC strain is ATCC H10407. Attenuated strains of enteropathogenic *E. coli* (EPEC), enteroinvasive *E. coli* (EIEC) or enterohemorrhagic *E. coli* (EHEC) may also be used.

Invasive bacteria are particularly useful, for embodiments of the invention where a eukaryotic expression cassette is used to drive expression of the heterologous antigen in the cells of the vaccinated individual. Examples of naturally invasive bacteria which may be employed include those from the genera *Salmonella, Shigella, Listeria* and *Rickettsia,* as well as ETEC.

Preferred *Shigella* strains for use in the invention include those described in US Patent 5,877,159. These include *Shigella flexneri* 2a (ATCC No. 29903), *Shigella sonnei* (ATCC No. 29930), and *Shigella disenteriae* (ATCC No. 13313). Attenuated *Shigella* strains include *Shigella flexneri* 2a 2457T ΔaroA.ΔvirG (Noriega et al, supra), *Shigella flexneri* M90T Δ*icsA* (Goldberg et al, Infect. Immun., 62:5664-5668 (1994)), *Shigella flexneri* Y SFLL114 *aroD* mutant (Karnell et al, Vacc., 10:167-174 (1992)), and *Shigella flexneri ΔaroAΔ. aroD* (Verma et al, Vacc., 9:6-9 (1991)).

Examples of non-naturally invasive bacteria which may be engineered to be invasive include those from the genera *Yersinia, Escherichia, Klebsiella, Bordetella, Neisseria, Aeromonas, Franciesella, Corynebacterium, Citrobacter, Chlamydia, Hemophilus, Brucella, Mycobacterium, Legionella, Rhodococcus, Pseudomonas, Helicobacter, Vibrio, Bacillus, Leishmania and Erysipelothrix.* These bacteria may be genetically engineered to mimic the invasion properties of *Salmonella, Shigella, Listeria, Rickettsia,* or enteroinvasive *E. coli.*

Typically, the bacterium is genetically engineered by inserting genes that enable it to access the cytoplasm of an animal cell. Examples of such genes include those encoding the invasive proteins of *Shigella,* hemolysin, those on the invasion plasmid of *Escherichia,* and listeriolysin O of *Listeria* (Formal et al, Infect. Immun., 46:465 (1984); Bielecke et al, Nature, 345:175-176 (1990); Small et al, In: Microbiology, 1986, 121-124, Levine et al, Eds., American Society for Microbiology, Washington, D.C. (1986); and Zychlinsky et al, Molec. Micro., 11:619-627 (1994)). Viral genes, such as influenza virus hemagglutinin HA-2, may also be used (Plank et al, J. Biol. Chem. , 269:12918-12924 (1994).

Any of the antigens mentioned herein can be expressed using the DNA vaccines of the invention. In particular, viral or eukaryotic antigens will be expressed as these will typically be optimized already for expression in eukaryotic cells. In the case of bacterial or non-eukaryotic antigens which are expressed poorly in eukaryotic cells due, for example, to particular codon usage the genes encoding these polypeptides may be modified to improve their expression in eukaryotic cells.

### Attenuating mutations

Preferably, the bacterium used in the invention is an attenuated pathogen. The attenuating mutation(s) employed generally knock-out the function of the gene(s) completely. This may be achieved either by abolishing synthesis of any polypeptide at all from the gene or by making a mutation that results in synthesis of a non-functional polypeptide. In order to abolish synthesis of any polypeptide, either the entire gene or its 5'-end may be deleted. A deletion or insertion within the coding sequence of a gene may be used to create a gene that synthesises only non-functional polypeptide (e.g. polypeptide that contains only the N-terminal sequence of the wild-type protein).

Preferably the mutations are non-reverting mutations. These are mutations that show essentially no reversion back to the wild-type when the bacterium is used as a vaccine. Such mutations include insertions and deletions. Insertions and deletions are preferably large, typically at least 10 nucleotides in length, for example from 10 to 600 nucleotides.

The bacterium used in the vaccine preferably contains only defined mutations, i.e. mutations which are characterised. It is undesirable to use a bacterium which has an uncharacterised mutation in its genome as a vaccine because there would be a risk that the uncharacterised mutation may confer properties on the bacterium that causes undesirable side-effects.

Attenuating mutations can be introduced into bacteria using non-specific mutagens either chemically, using agents such as N-methyl-N'-nitro-N-nitrosoguanidine, or using recombinant DNA techniques, such as Tn10 mutagenesis, P22-mediated transduction, lambda phage mediated crossover, and conjugational transfer. However, site-directed mutagenesis using recombinant DNA techniques is preferred since strains constructed in this way are far more defined. In particular the deletion or mutation of genes by techniques involving homologous recombination is preferred.

Examples of attenuating mutations include, but are not limited to:
(i) auxotrophic mutations, such as *aro* (Hoiseth et al, Nature, 291:238-239 (1981)), *gua* (McFarland et al, Microbiol. Path., 3:129-141 (1987)), nad (Park et al, J. Bact., 170:3725-3730 (1988), *thy* (Nnalue et al, Infect. Immun., 55:955-962 (1987)), and *asd* (Curtiss, supra) mutations;
(ii) mutations that inactivate global regulatory functions, such as *cya* (Curtiss et al, Infect. Immun., 55:3035-3043 (1987)), *crp* (Curtiss *et al* (1987), supra), *phoP*/*phoQ* (Groisman et al, Proc. Natl. Acad. Sci., USA, 86:7077-7081 (1989); and Miller et al, Proc. Natl. Acad. Sci., USA, 86:5054-5058 (1989)), *phoP.sup.c* (Miller et al, J. Bact., 172:2485-2490 (1990)) or *ompR* (Dorman et al, Infect. Immun., 57:2136-2140 (1989)) mutations;
(iii) mutations that modify the stress response, such as *recA* (Buchmeier et at, Mol. Micro., 7:933-936 (1993)), *htrA* (Johnson et at, Mol. Micro., 5:401-407 (1991)), *htpR* (Neidhardt et al, Biochem. Biophys. Res. Com., 100:894-900 (1981)), *hsp* (Neidhardt et at, Ann. Rev. Genet., 18:295-329 (1984)) and *groEL* (Buchmeier et at, Sci., 248:730-732 (1990)) mutations;
(iv) mutations in specific virulence factors, such as *lsyA* (Libby et al, Proc. Natl. Acad. Sci., USA, 91:489-493 (1994)), *pag* or *prg* (Miller *et al* (1990), supra; and Miller *et al* (1989), supra), *iscA* or *virG* (d'Hauteville et al, Mol. Micro., 6:833-841 (1992)), picA (Mengaud et al, Mol. Microbiol., 5:367-72 (1991); Camilli et al, J. Exp. Med, 173:751-754 (1991)), and act (Brundage et al, Proc. Natl. Acad. Sci., USA, 90:11890-11894 (1993)) mutations;
(v) mutations that affect DNA topology, such as *topA* (Galan et al, Infect. Immun., 58:1879-1885 (1990)) mutation;
(vi) mutations that block biogenesis of surface polysaccharides, such as *rfb, galE* (Hone et al, J. Infect. Dis., 156:164-167 (1987)) or via (Popoff et al, J. Gen. Microbiol., 138:297-304 (1992)) mutations;
(vii) mutations that modify suicide systems, such as *sacB* (Recorbet et al, App. Environ. Micro., 59:1361-1366 (1993); Quandt et al, Gene, 127:15-21 (1993)), nuc (Ahrenholtz et al, App. Environ. Micro., 60:3746-3751 (1994)), *hok, gef, kil,* or *phLA* (Molin et al, Ann. Rev. Microbiol., 47:139-166 (1993)) mutations;
(viii) mutations that introduce suicide systems, such as lysogens encoded by P22 (Rennell et al, Virol., 143:280-289 (1985)), lambda murein transglycosylase (Bienkowska-Szewczyk et al, Mol. Gen. Genet., 184:111-114 (1981)) or S-gene (Reader et al, Virol., 43:623-628 (1971)); and
(ix) mutations that disrupt or modify the correct cell cycle, such as *minB* (de Boer et al, Cell, 56:641-649 (1989)) mutation.

Preferably, the bacterium used in the invention is attenuated by a non-reverting mutation in one, two, three or four separate genes. Typically the mutation will be in at least one of the following genes: an *aro* gene, *a pur* gene, *ompC, ompF, ompR, htrA, galE, cya, crp, and phoP.*

In a preferred embodiment of the invention the bacterium is attenuated by a non-reverting mutation in:
- the *htrA* gene and at least one *aro* gene;
- at least one *aro* gene and at least one *omp* gene;
- each of the *aroc* gene, the *ompF* gene and the *ompC* gene; or
- each of two discrete *aro* genes, in particular *aroA* and *aroC, aroA* and *aroD* or *aroc* and *aroD.*

### Heterologous Polypeptides

A wide variety of heterologous polypeptides may be expressed by the bacteria used in the vaccines of the invention. The polypeptide is "heterologous" to the bacterium in the sense that it is not naturally expressed by the bacterium. The polypeptide is typically from a different species to the host bacterium, but may be from a different strain of the same species. The bacterium may be engineered to express more than one heterologous polypeptide, in which case the polypeptides may be from the same organism or from different organisms.

In a preferred embodiment of the invention, the heterologous polypeptide is a heterologous antigen of a pathogen. Two or more heterologous antigens from different pathogens may be expressed allowing immunisation against each of the separate pathogens with a single vaccine.

The heterologous polypeptide may be a complete protein or a part of a protein containing an epitope. In one embodiment of the invention, the heterologous polypeptide is a fusion protein. The fusion may involve two or more different antigens or an antigen and a region designed to increase the immunogenicity of the heterologous polypeptide. Fusions with the atoxic fragment C from tetanus toxin (TetC) may be used.

The antigen may be from a bacterium, a virus, a yeast, a fungus or a parasite.

Examples of viruses the heterologous antigen may be derived from include: Respiratory syncytial virus; Orthomyxoviruses, such as influenza virus; Retroviruses, such as RSV, HIV and SIV; Herpesviruses, such as EBV, CMV or herpes simplex virus; Lentiviruses, such as human immunodeficiency virus; Rhabdoviruses, such as rabies; Picornoviruses, such as poliovirus; Poxviruses, such as vaccinia; Rotaviruses; and Parvoviruses.

Examples of preferred viral antigens to be expressed as the heterologous antigen include the human immunodeficiency virus antigens Nef, p24, gp120, gp41, Tat, Rev, and Pol (Nature, 313:277-280 (1985)) and T cell and B cell epitopes of gp120 (Palker et al, J. Immunol., 142:3612-3619 (1989)); the hepatitis B surface antigen (Wu et al, Proc. Natl: Acad. Sci., USA, 86:4726-4730 (1989)); rotavirus antigens, such as VP4 (Mackow et al, Proc. Natl. Acad. Sci., USA, 87:518-522- (1990)) and VP7 (Green et al, J. Virol., 62:1819-1823 (1988)), influenza virus antigens such as hemagglutinin or nucleoprotein (Robinson *et al.,* Supra; Webster *et al,* Supra) and herpes simplex virus thymidine kinase (Whitley et al, In: New Generation Vaccines, pages 825-854).

Examples of pathogenic bacteria from which the heterologous antigen may be derived include bacteria of the genera *Mycobacterium, Haemophilus, Bordetella Helicobacter, Salmonella, Shigella, Escherichia, Rickettsia, Listeria. Legionella, Pseudomonas, Yersinia, Vibrio, Borellia, Lactococcus, Lactobacillus, Brucella, Aeromorcas, Franciesella, Corynebacterium, Citrobactor, Rhodococcus, Leishmania, Neisseria* and *Chlamydia.*

Examples of preferred bacterial antigens to be expressed as the heterologous antigen include the *Shigella sonnei* form 1 antigen (Formal et al, Infect. Immun., 34:746-750 (1981)); the F1 antigen of *Yersinia pestis* (Titball, et al, Infect Immun. 1997, 65(5), 1926-1930); antigens from *Neisseria meningititidis* and in particular those encoded by the GNA33, GNA2001, GNA1220 and GNA1946 genes (Pizza et al, Science, 2000, 287, 1816 to 1820 (2000)); the O-antigen of *V. cholerae* Inaba strain 569B (Forrest et al, J. Infect., Dis., 159:145-146 (1989); protective antigens of enterotoxigenic *E.coli,* such as fimbrial antigens including colonisation factor antigens, in particular CFA/I, CFA/II, and CFA/IV (Yamamoto et al, Infect. Immun., 50:925-928 (1985)) and the nontoxic B-subunit of the heat-labile toxin (Clements et al, 46:564-569 (1984)); pertactin of *Bordetella pertussis* (Roberts et al, Vacc., 10:43-48 (1992)), adenylate cyclase-hemolysin of *B. pertussis* (Guiso et al, Micro. Path., 11:423-431 (1991)); fragment C of tetanus toxin of *Clostridium tetani* (Fairweather et al, Infect. Immun., 58:1323-1326 (1990)) and the LT (heat labile enterotoxin) and ST (heat stable toxin) antigens.

Examples of parasitic pathogens the heterologous antigen may be derived from include those from the genera *Plasmodium, Chtamydia, Trypanosome, Giardia, Boophilus, Babesia, Entamoeba, Eimeria, Leishmania, Schistosome, Brugia, Fascida, Dirofilaria, Wuchereria* and *Onchocerea.*

Examples of preferred antigens from parasitic pathogens to be expressed as the heterologous antigen include the circumsporozoite antigens of *Plasmodium* species (Sadoff et al, Science, 240:336-337 (1988)), such as the circumsporozoite antigen of *P. bergerii* or the circumsporozoite antigen of *P. falciparum;* the merozoite surface antigen of *Plasmodium* species (Spetzler et al, Int. J. Pept. Prot. Res., 43:351-358 (1994)); the galactose specific lectin of *Entamoeba histolytica* (Mann et al, Proc. Natl. Acad. Sci., USA, 88:3248-3252 (1991)); gp63 of *Leishmania* species (Russell et al, J. Immunol., 140:1274-1278 (1988)); paramyosin of *Brugia malayi* (Li et al, Mol. Biochem. Parasitol., 49:315-323 (1991)); the triose-phosphate isomerase of *Schistosoma mansoni* (Shoemaker et al, Proc. Natl. Acad. Sci., USA, 89:1842-1846 (1992)); the secreted globin-like protein of *Trichostrongylus colubriformis* (Frenkel et al, Mol. Biochem. Parasitol., 50:27-36 (1992)); the glutathione-S-transferases of *Frasciola hepatica* (Hillyer et al, Exp. Parasitol., 75:176-186 (1992)), *Schistosoma bovis* and *S. japonicum* (Bashir et al, Trop. Geog. Med., 46:255-258 (1994)); and KLH of *Schistosoma bovis* and *S. japonicum* (Bashir *et al,* supra).

The heterologous antigen may be a tumour specific antigen. Examples of preferred tumour specific antigens include prostate specific antigen (Gattuso et al, Human Pathol., 26:123-126 (1995)), TAG-72 and CEA (Guadagni et al, Int. J. Biol. Markers, 9:53-60 (1994)), MAGE-1 and tyrosinase (Coulie et al, J. Immunothera., 14:104-109 (1993)).

The heterologous antigen may be a transplant antigen. Examples of preferred transplant antigens include the CD3 receptor on T cells (Alegre et al, Digest. Dis. Sci., 40:58-64 (1995)).

The heterologous antigen may be a modified or native self antigen from the species to be immunised. The heterologous antigen may be an autoimmune antigen. Examples of autoimmune antigens include IASβ chain (Topham et al, Proc. Natl. Acad. Sci., USA, 91:8005-8009 (1994)).

In a particularly preferred embodiment of the invention the heterologous antigen is derived from a bacterium of the genus *Helicobacter* and in particular from *Helicobacter pylori.* The *Helicobactor pylori* antigen may be urease, catalase, AlpA or a fragment of any of these. In a preferred embodiment of the invention, two or more *H. pylori* antigens are provided by the vaccine.

In some embodiments of the invention the heterologous polypeptide encoded by the plasmid to be stabilised may be other than, or in addition to, sequences encoding a heterologous antigen. For example, the polypeptide may regulate or turn on expression of the heterologous antigen encoded by sequences on the bacterial chromosome or a second plasmid. Alternatively, or in addition, the heterologous polypeptide encoded by the plasmid may be a selection marker or a polypeptide required for optimal growth of the bacterium carrying the plasmid.

In the case of the heterologous polypeptide playing a regulatory role it may bind to and activate, or increase expression from, the sequences encoding the heterologous antigen. The regulation may be inducible so that expression of the antigen is only activated at an appropriate time, for example when the bacteria are at an appropriate stage of growth or administered to the host to be vaccinated. This may help avoid, or reduce, early selection pressure against bacteria carrying the plasmid until expression is induced.

### Formulation of the vaccine

The vaccine may be formulated using known techniques for formulating attenuated bacterial vaccines. The vaccine is typically presented for oral administration, for example as a dried stabilised powder for reconstitution in a buffer prior to administration. Reconstitution is advantageously effected in a buffer at a suitable pH to ensure the viability of the bacteria. In order to protect the attenuated bacteria and the vaccine from gastric acidity, a sodium bicarbonate preparation is advantageously administered with each administration of the vaccine. Alternatively, the vaccine is presented in a hypophilised encapsulated form.

The vaccine may be used in vaccination, preferably of a mammalian host, particularly a human host but also an animal host. An infection caused by a microorganism, especially a pathogen, may therefore be prevented by administering an effective dose of a vaccine prepared according to the invention. In one embodiment of the invention the vaccine will reduce the load of pathogenic organism or alternatively not prevent infection but prevent or ameliorate a disease resulting from infection. The dosage employed may ultimately be at the discretion of the physician, and may be dependent on various factors including the size and weight of the host and the type of vaccine formulated. However, a dosage comprising the oral administration of from 10⁷ to 10¹¹ bacteria, preferably from 10⁸ to 10⁹ and more preferably from 5x10⁸ bacteria per dose may be convenient for a 70 kg adult human host.

In some embodiments of the invention the vaccine may comprise more than one bacterial strain or species, wherein each bacterium expresses a different antigen or set of antigens.

### Examples

### Brief Description of the Drawings

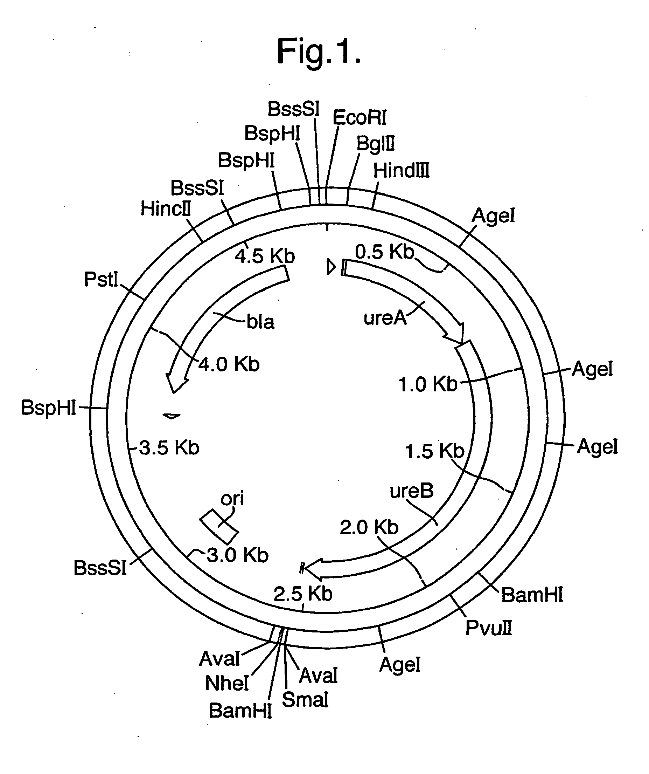

**Figure 1****.** Genetic map of plasmid pHUR3 which uses the origin of replication and ampicillin determinant of plasmid pBR322 and the *UreAB* genes derived from *Helicobacter pylori* and expressed from the *Salmonella typhimurium htrA* promoter.

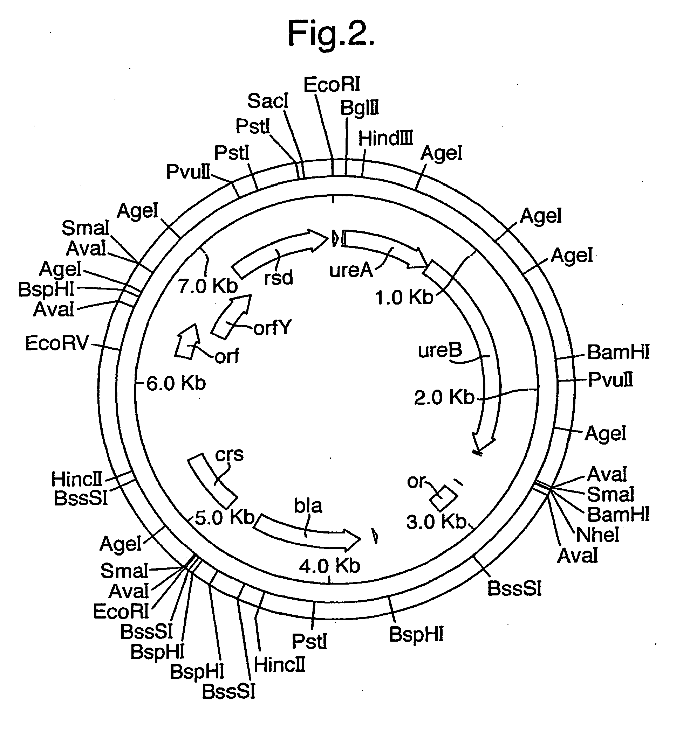

**Figure 2****.** Genetic map of plasmid pHUR3rsd which was generated from pHUR3 by insertion of a 3 kb fragment carrying the *trans* acting resolvase gene (*rsd*) and the *cis* acting multimer resolution site (*crs*) from the virulence plasmid of *Salmonella dublin.*

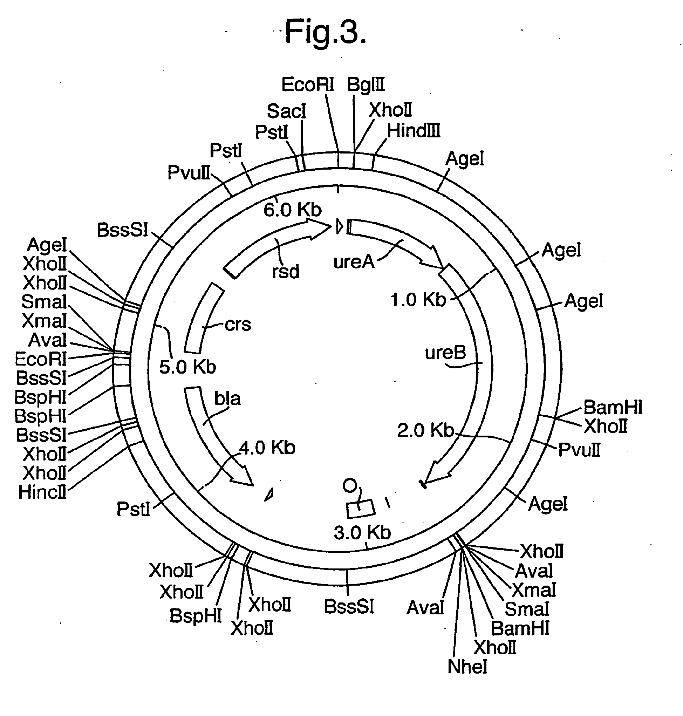

**Figure 3****.** Genetic map of plasmid pHUR*3rsd*Δ⁵⁹⁷⁴ which was generated from pHUR3 by insertion of a 1.5 kb cassette carrying the *rsd* gene and the *crs* site but lacking the intervening sequences between the two which are present in the virulence plasmid of *Salmonella dublin.*

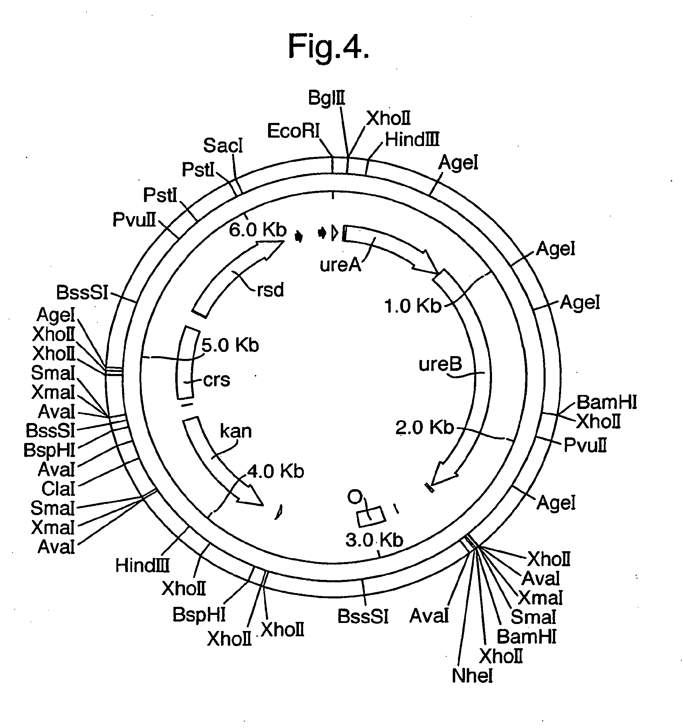

**Figure 4****.** Genetic map of plasmid pH3RC3 which was generated from pHUR3rsdΔ⁵⁹⁷⁴ by insertion of the 3' region of the *rrnB* 5S RNA gene of *E. coli,* which acts as a transcriptional terminator, immediately upstream of the *htrA* promoter.

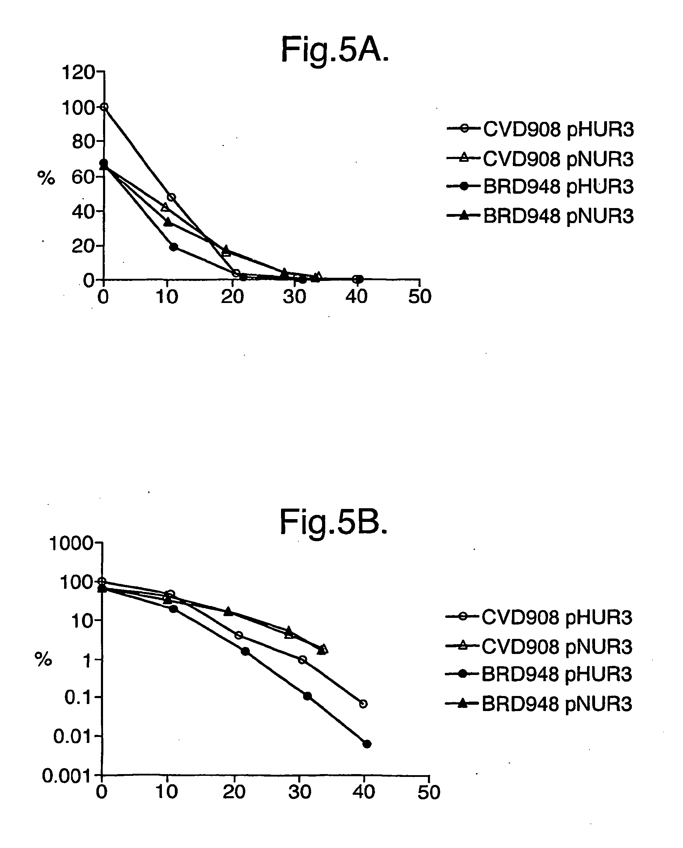

**Figure 5****.** Inheritance of plasmids pHUR3 and pNUR3 in *Salmonella typhi* strains CVD908 and CVD948 shown as the proportion of bacteria retaining the plasmid against the number of generations undergone. (A) Shows the percentage of bacteria retaining the plasmid against the number of generations. (B) Shows the percentage of bacteria retaining the plasmid (on a log scale) against the number of generations (on a linear scale).

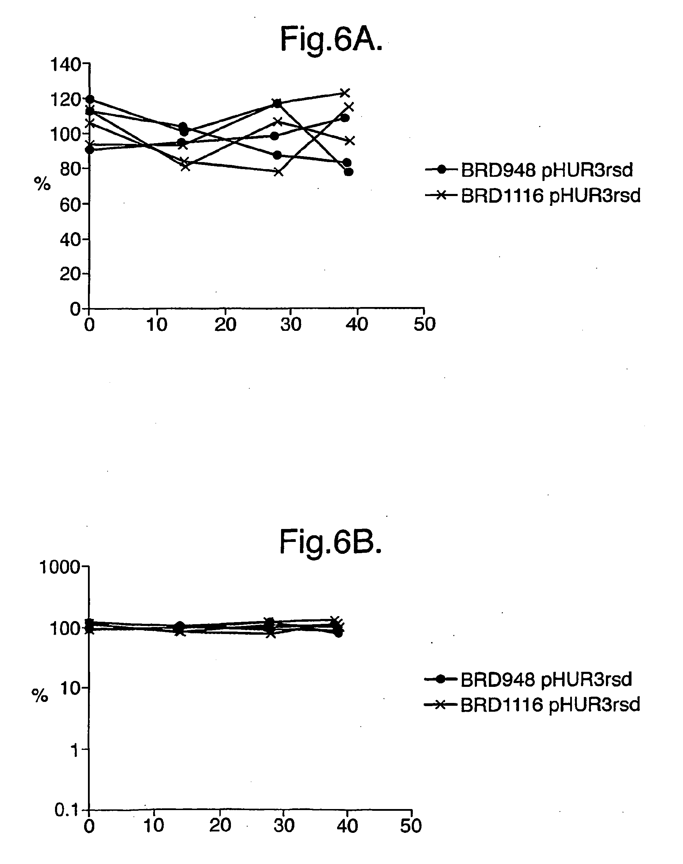

**Figure 6****.** Inheritance of plasmid pHUR3rsd in *Salmonella typhi* strains BRD948 and BRD1116 over time. (A) Shows the percentage of bacteria retaining the plasmid against the number of generations. (B) Shows the percentage of bacteria retaining the plasmid (on a log scale) against the number of generations (on a linear scale).

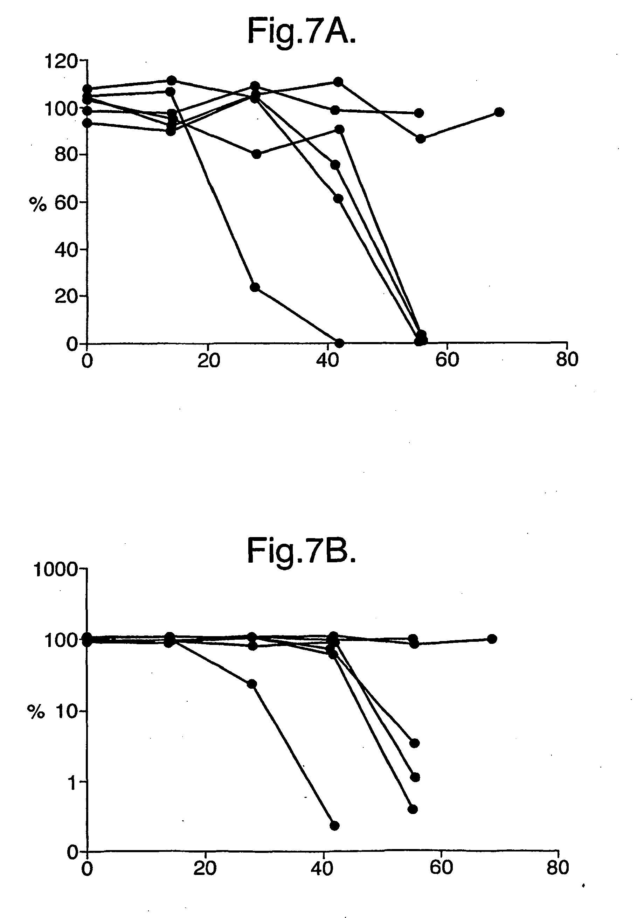

**Figure 7****.** Inheritance of plasmid pHUR3rsdΔ⁵⁹⁷⁴ in *Salmonella typhi* strain BRD948. (A) Shows the percentage of bacteria retaining the plasmid against the number of generations. (B) Shows the percentage of bacteria retaining the plasmid (on a log scale) against the number of generations (on a linear scale).

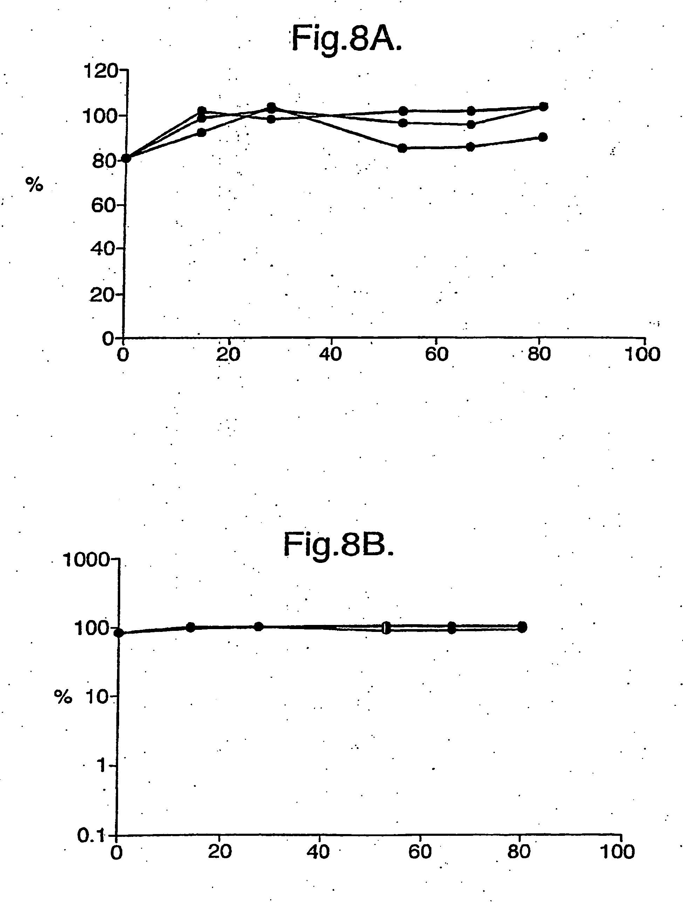

**Figure 8****.** Inheritance of plasmid pH3RC3 in *Salmonella typhi* strain BRD948. (A) Shows the percentage of bacteria retaining the plasmid against the number of generations. (B) Shows the percentage of bacteria retaining the plasmid (on a log scale) against the number of generations (on a linear scale).
**Figure 9****.** Effect of the Rsd/crs system on the pH3RC3 multimer resolution. Plasmid DNA from BRD948 harboring pHUR3 (a control plasmid) and pH3RC3 (a plasmid in accordance with the invention) was extracted and subjected to agarose gel electrophoresis. Plasmid pHUR3 was less mobile than pH3RC3, despite the latter being a larger plasmid. This suggests that pHUR3 is multimerised compared to pHRC3 in *Salmonella typhimurium* strain BRD948, reinforcing the notion that the instability of inheritance observed for pHUR3 is due to formation of plasmid multimers.

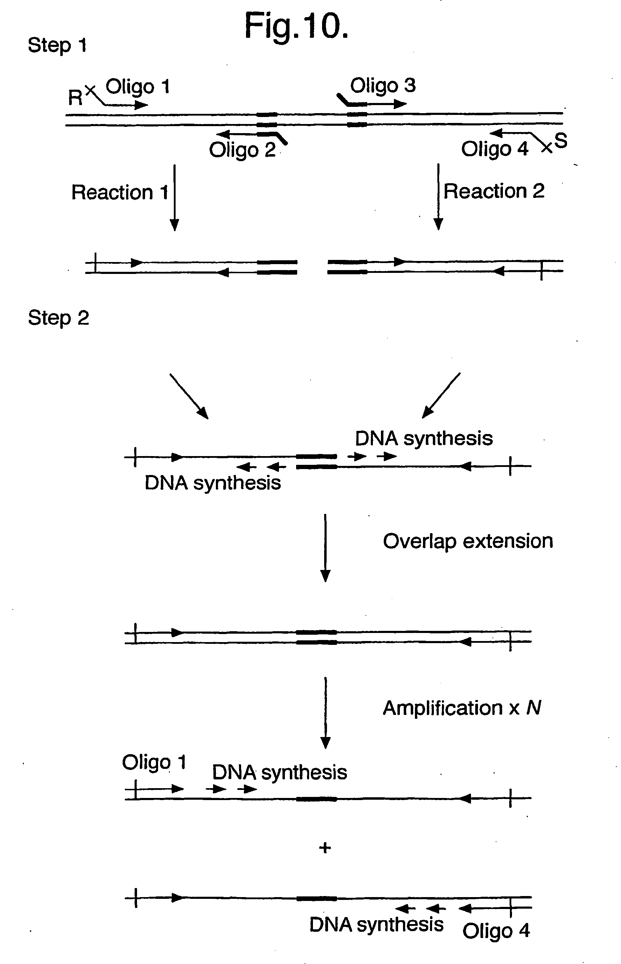

**Figure 10**. Illustration of modified overlap extension PCR. Modified overlap extension PCR may be used to generate defined deletion mutations in DNA fragments. Four oligonucleotides are designed to amplify two DNA fragments that flank the region to be deleted. The outermost oligonucleotides (Oligo 1 and Oligo 4) would normally include restriction endonuclease sites in their 5'-ends ("R" and "S") to allow cloning of the final product. About 20 bases of the 5'-ends of the inner oligonucleotides (Oligo 2 and Oligo 3) are complementary to each other (represented by bold lines) to allow based pairing of the ends of the resulting DNA fragments. In Step 1, the two DNA fragments that flank the region to be deleted are generated in separate PCR reactions using Oligo 1 with Oligo 2, and Oligo 3 with Oligo 4. In step 2 these DNA fragments so generated are used as the template in another PCR reaction using Oligo 1 with Oligo 4.

### Brief Description of the sequences

SEQ ID NO: 1 shows the nucleotide sequence of a cassette usable in the invention comprising the *rsd* and *crs* sequences (with the naturally occurring intervening sequence removed) and *rrnB* transcription termination elements.
SEQ ID NO: 2 shows the amino acid sequence of the Rsd recombinase encoded in the sequence of SEQ ID NO: 1.
SEQ ID NO: 3 shows the nucleotide sequence of PCR primer 5959.
SEQ ID NO: 4 shows the nucleotide sequence of PCR primer 5960.
SEQ ID NO: 5 shows the nucleotide sequence of PCR primer 5961.
SEQ ID NO: 6 shows the nucleotide sequence of PCR 5962.
SEQ ID NO:7 shows the nucleotide sequence of PCR primer 5974.
SEQ ID NO: 8 shows the nucleotide sequence of PCR primer 5989.
SEQ ID NO: 9 shows the nucleotide sequence of PCR primer 5990.
SEQ ID NO: 10 shows the nucleotide sequence of PCR primer 5991.
SEQ ID NO: 11 shows the nucleotide sequence of PCR primer 5992.

### Materials and Methods

**Bacterial strains and plasmids. Bacterial strains and plasmids used are listed in Table 1.**

Recombinant DNA techniques. Standard procedures were used (Sambrook et al. Molecular cloning: a Laboratory Manual, 2nd Edition, CSH Laboratory Press, 1989). Plasmid purification was performed using kits from QIAGEN^{™}. PCR amplification of DNA fragments for cloning was performed using the high-fidelity *Pfu* Turbo^{™} polymerase (Stratagene^{™}). Other PCR amplification was performed using Taq DNA polymerase (Life Technologies^{™}). DNA fragments were isolated from agarose gels using the QIAquick gel extraction kit from QIAGEN^{™}.

Plasmid inheritance assay. L-broth cultures of *Salmonella typhi* strains supplemented with ampicillin at 200 mg/ml, carbenicillin at 300mg/ml, or kanamycin at 20 ing/ml were inoculated from a colony and grown at 37°C overnight. This culture was used to inoculate fresh L-broths at a dilution of 1:10,000 without antibiotic selection, and the new culture grown overnight at 37°C. The strain was passaged like this several times and, for each culture, dilutions were plated in triplicate onto L-agar and L-agar supplemented with ampicillin at 200 mg/ml or kanamycin at 20 mg/ml in order to determine the total numbers and numbers of antibiotic resistant bacteria. The stability of each plasmid as measured by the proportion of colonies remaining antibiotic resistant was determined in at least 3 independent experiments. Data in Figures 5,- 8 are plotted as the mean of triplicate determinations at each time point.

**Western blot analysis for urease expression.** This was performed using apparatus and reagents from Novex^{™}. Bacterial cells were harvested from 1 ml of an L-broth culture incubated overnight at 37°C. Cells were washed in 1 ml PBS and resuspended in 100ml PBS. An equal volume of SDS-PAGE sample buffer supplemented with 2 % β-mercaptoethanol was added and the samples incubated for 5 mins in a boiling water bath. Volumes of 3 to 6ml were electrophoresed through 12% polyacrylamide gels and the proteins were then electro-transferred to nitrocellulose membranes. Subsequent treatments were performed at room temperature. Membranes were blocked with 5 % skimmed milk in PBS, 0.05 % Tween 20^{™} (PBST) for 1 h then transferred to anti-urease polyclonal rabbit serum diluted 1:2,000 in 1 % skimmed milk PBST for 1 h. Membranes were then washed 4 times for 10 mins in 1 % skimmed milk PBST, then incubated in goat anti-rabbit IgG-HRP conjugate diluted 1:2,000 in 1 % skimmed milk PBST for 1 h. Membranes were washed 4 times as described previously then added to enhanced chemiluminescence (ECL) detection reagents (Amersham^{™}) before being exposed to ECL film.

### Results

### Example 1:

**Plasmid loss in** ***S. typhi.*** Plasmids pHUR3 (Figure 1) and pNUR3 use the origin of replication and ampicillin resistance determinant of pBR322 and the *ureAB* genes derived from *Helicobacter pylori* expressed from the *Salmonella typhimurium htrA* or *nirB* promoter respectively. We have observed that both plasmids are lost from *Salmonella typhi* vaccine strains CVD908 and BRD948 during exponential growth in broth cultures. The instability is such that after 30 generations generally less than 20% of the bacterial cells continue to harbour the recombinant plasmid. In addition, even in the presence of selection with ampicillin a significant proportion of the bacteria in stationary phase cultures do not carry the plasmid (Figure 5). This is probably because ampicillin is rapidly inactivated by the large numbers of bacteria expressing β-lactamase in these cultures.

**Construction of plasmid derivatives incorporating the *rsd-crs* locus from the *S. dublin* virulence plasmid.** The trans-acting resolvase gene (*rsd*) and the *cis-*acting multimer resolution site (*crs*) from the virulence plasmid of *Salmonella dublin* have been mapped within a 3 kb fragment generated by the restriction endonucleases EcoRV and *Sma*I, and their nucleotide sequences determined (Krause et al. J. Bacteriol 1991. 173(18): 5754-5762). A fragment of DNA incorporating the *rsd-crs* locus was amplified from plasmid DNA prepared from *Salmonella dublin* using oligonucleotides 5960 and 5961 as primers. This fragment was digested with *EcoRI* and cloned into the unique *EcoR*I site of pHUR3 to give plasmid pHUR3rsd (Fig2). This plasmid was used to transform the *Salmonella typhi* strains BRD948 and BRD1116 by electroporation. Both of the resulting strains showed significantly increased stability of inheritance as compared to strains harbouring plasmid pHUR3 (Figure 6).

### Example 2:

Plasmid pHUR3rsd incorporates the *rsd* recombinase and the *crs* recombination site from the *Salmonella dublin* virulence plasmid in their natural configuration, with an intervening sequence of approximately 1.5kb including open reading frames of unknown function. We wished to minimise the size of the recombinant expression vector and so generated a variant of pHUR3*rs*d in which this intervening sequence was removed. The *rsd* and *crs* loci were separately amplified by PCR using a plasmid DNA preparation from *Salmonella dublin* as template and oligonucleotides 5960 and 5962 for *crs,* and 5961 and 5974 for *rsd* (Table 2) as primers. The two fragments were then spliced by overlap extension PCR and the resulting *rsd-crs* cassette cloned into the vector pPCR-Script (Stratagene^{™}). From here the cassette was sub-cloned into the *EcoRI* site of pHUR3 to give plasmid *pHUR3rsdΔ⁵⁹⁷⁴* (Figure 3).

**The *rsd-crs* cassette stabilises inheritance of *pHUR3rsdΔ⁵⁹⁷⁴* in attenuated *Salmonella typhi* strains.** Plasmid *pHUR3rsdΔ⁵⁹⁷⁴* was transformed into the attenuated *Salmonella typhi* strain BRD948 and the proportion of cells harbouring the plasmid was measured over a number of generations in several independent experiments. The pHUR3*rsdΔ*⁵⁹⁷⁴ derivative was significantly more stable than the parent plasmid pHUR3 in most experiments (Figure 7). There was, however, some variation in the rate of loss of pHUR3 *rsdΔ⁵⁹⁷⁴* between experiments. In addition, once loss of pHITR3*rsd*Δ⁵⁹⁷⁴ was initiated, it was lost at a faster rate than pHUR3.

**UreAB is over-expressed from pHUR3*rsd*Δ⁵⁹⁷⁴.** Plasmid pHUR3 is an expression vector in which the urease, *ureAB,* operon from *Helicobacter pylori* is expressed from the *Salmonella htrA* promoter. This ensures that expression of *ureAB* is induced when the *Salmonella typhi* invades the host tissues which has been found to enhance immune responses against heterologous antigens. In addition, expression of heterologous antigens can have a destabilising influence on the inheritance of the plasmids by which they are encoded. It is therefore desirable to repress expression of heterologous antigens during *in vitro* growth to help maintain their encoding plasmid. We therefore wanted to confirm that introduction of the *rsd-crs* cassette into pHUR3 had not affected expression of *ureAB.* Western blot analysis of *Slamonella Typhi* strains indicated that expression of UreAB from pHUR*3rsd*Δ⁵⁹⁷⁴ was significantly higher than from pHUR3. This was also the case when the orientation of the *rsd-crs* cassette was reversed. This suggests that there was strong and divergent promoter activity initiated within the *rsd-crs* cassette, leading to uncontrolled transcription of the *ureAB* operon.

We conclude that the plasmid pHUR*3rsd*Δ⁵⁹⁷⁴ confers a significant burden upon *Salmonella typhi* cells which contain it due to the high level of expression of urease due to the additional unregulated promoter in the stabilising cassette. When rare cells arise from which the plasmid has been lost they outgrow the cells which continue to express urease at a faster rate due to this additional burden. Thus the kinetics of plasmid loss once it has started is faster for pHUR3rsdΔ⁵⁹⁷⁴ than for pHUR3.

### Example 3:

The 3'-region of the *rrnB* 5S RNA gene of *E. coli* incorporates a strong transcriptional terminator (Brosius et al. Gene, 1984. 27(2): 161-172). In order to counter the effects of the *rsd-crs* cassette on urease expression a pHUR3*rsd*Δ⁵⁹⁷⁴ derivative was constructed with this transcriptional terminator immediately upstream of the *htrA* promoter. In addition, because β-lactam antibiotics are frequently used in therapy it is preferable not to have β-lactamase resistance determinants as a component of vaccine strains. We therefore replaced the *bla* gene of this pHUR3*rsd*Δ⁵⁹⁷⁴ derivative with a kanamycin resistance determinant to give plasmid pH3RC3 (Figure 4).

Plasmid pH3RC3 was constructed in the following way. Firstly, the *Eco*RI site proximal to *crs* was deleted. This was achieved by isolating linearised plasmid from an agarose gel following partial digestion with *Eco*RI*.* The cohesive ends were filled and the DNA ligated and transformed into *E. coli* strain XL10-Gold (Stratagene^{™}). A recombinant plasmid with the relevant *Eco*RI site deleted was selected and called pH3RC1. The *rrnB* 5S transcritional terminator region was PCR amplified from *E. coli* DNA using the oligonucleotides 5991 and 5992 (Table 2) as primers. By virtue of the oligonucleotides, the resulting fragment includes an *EcoRI* site at one end and an *ApoI* site, which generates a cohesive end compatible with *EcoRI,* at the other. The fragment resulting from PCR amplification was cloned into pPCR-Script (Stratagene^{™}) and from there cleaved out using *Apol* and subcloned into the *Eco*RI site of pH3RC1. A recombinant plasmid with the cloned fragment in the appropriate orientation was identified by PCR using oligonucleotides 5992 and 5959 as primers and called pH3RC2. The kanamycin resistance determinant was PCR amplified from the kanamycin GenBlock (from Pharmacia^{™}) using oligonucleotides 5989 and 5990 (Table 2) as primers. The resulting fragment was digested with *Bsp*HI and cloned directly into pH3RC2 digested with the same restriction endonuclease to remove the ampicillin resistance determinant. Kanamycin resistant derivatives were selected and the orientation of the kanamycin resistance determinant was confirmed by PCR using oligonucleotides 5962 and 5990 which generated a DNA fragment of the expected size (approximately 1.2kb) in correct constructs. One of these derivatives was called pH3RC3 (Figure 4).

**In vitro stability of plasmid pH3RC3 in** ***S.typhi.*** Plasmid pH3RC3 was introduced into BRD948 and assayed for inheritance over a large number of generations. It was maintained without loss for 80 generations in three experiments performed independently (Figure 8).

### Example 4:

The present invention can also be used in stabilising DNA vaccines, and this Example is a paper Example to illustrate how DNA vaccines can be made.

The *crs-rsd* cassette can be excised from pH3RC3 on a restriction fragment and inserted into a DNA vaccine vector by standard cloning methods. For example, pVAX-F can be generated from the commercially available vector pVAX-1 (Invitrogen^{™}) by cloning the gene encoding the F antigen of respiratory syncytial virus on an EcoRI fragment into the multiple cloning site downstream of the CMV-β promoter.

The stability of the DNA vaccines of the invention can be assessed in *Salmonella typhi* and *Salmonella typhimurium* using the same *in vitro* system as described for the prokaryotic expression plasmids. In addition, the *Salmonella* strains harboring the plasmid could be allowed to infect macrophage cell lines such as J774 and U937 in culture and the ability of the strains to retain plasmids over time determined.

*Salmonella typhimurium* strains containing the DNA vaccines can be used to vaccinate mice orally. The persistence of the strains, and the stability of the plasmid within them, can be determined in spleens from the mice at various time points following vaccination.

### Example 5:

The *crs* region of the *crs-rsd* cassette has strong promoter activity in both directions. This has been demonstrated by the increased levels of urease expression from plasmid pHLTR3*rsd*Δ⁵⁹⁷⁴ compared to pHUR3. This property is undesirable in expression vectors such as those which are designed to give controlled expression *in vivo* from eg. the *htrA* or *nirB* promoter. The promoter activity of the *crs* region may be distinct from the sites required for Rsd binding and recombination which give rise to the stable inheritance characteristic. Mapping of the *crs* region to determine the promoter sites and those required for recombination could allow construction of derivatives that have the recombination site activity but not the promoter activity. This is a paper Example of how this could be done.

Deletion derivatives of the *crs* region can be generated using a modification of overlap extension PCR described previously (see Figure 10 and Tao, B.Y. and K.C.P. Lee, Mutagenesis by PCR, in PCR Technology: current innovations, H.G. Griffin and A.M. Griffin, Editors. 1994, CRC Press, Inc.: Boca Raton, Florida. p. 69-83). To achieve this, DNA fragments flanking the region to be deleted are generated in two separate reactions by PCR using four oligonucleotides (Oligos 1 & 2 and Oligos 3 & 4 in Figure 10). The two oligonucleotides immediately flanking the region to be deleted (Oligo 2 and Oligo 3 in Figure 10) have 5'-nucleotide sequences of approximately 20 bp which are complementary to each other. When the two fragments generated in this way are mixed and used as the template in a third PCR reaction which incorporates the outside oligonucleotides (Oligo 1 and Oligo 4 in Figure 10) as primers, a single *crs* deletion fragment is generated.

Stabilising recombination activity can be determined as follows. A derivative of plasmid pH3RC3 can be generated in which the *BssSI* restriction endonuclease site near the replication origin has been deleted. This may be done by partial digestion of plasmid pH3RC3 with *Bss*SI*,* filling in the cohesive ends with the Klenow fragment of DNA polymerase, ligating and transforming into a laboratory strain of *E. coli.* The required recombinant plasmid can then be found from plasmid preparations digested with *BssSI.* The desired plasmid gives *BssSI* fragments of approximately 6 kb and 0.6 kb. Deletion fragments can be generated by extension PCR using pH3RC3 in the first pair of PCR reactions. Thus the oligonucleotides can be manufactured so as to hybridise outside of the *Bss*SI fragment of plasmid pH3RC3 that includes *crs.* The incorporated *Bss*SI sites can then be used to clone the deletion fragment in place of the wild-type *crs* fragment of plasmid pH3RC3 and the heritability of these plasmid derivateves can be tested using the plasmid inheritance assay described in Materials and Methods. Any plasmid derivatives that show significant loss can be deemed to have had deleted the element(s) required for stabilising recombination.

Promoter activity may be measured by incorporating the deletion fragments in front of *gfp,* a gene which codes for the green fluorescence protein, carried by a plasmid expression vector such as pFPV25 described by Valdivia and Falkow (Bacterial genetics by flow cytometry: rapid isolation of Salmonella typhimurium acid-inducible promoters by differential fluoresence induction. Mol Microbiol, 1996. 22(2): p. 367-78). For this purpose oligonucleotides which hybridise to the outside of the *crs* region would be required to include *Bam*HI or *Bg1*II restriction enzyme sites to facilitate incorporation into the *Bam*HI site of pFPV25 which is the location required to drive expression of the *gfp* gene.

Once the locations of the stabilising recombination activity and promoter activity have been determined approximately, it may be possible to delete the promoter activity without losing the recombination activity.

If it is found that the recombination and promoter activities are located within the same region, or there is significant overlap, it may nevertheless be possible to inactivate the promoter activity by introducing point mutations which do not affect the recombination activity. This may be done using overlap extension PCR in the same way as described by Tao and Lee (1994) in the paper cited above. The mutant fragments can then be assayed for recombination activity using the plasmid stability assay, and for promoter activity in the *gfp* expression vector assay mentioned above.

**Table 1. Bacterial strains and plasmids**

| **Bacterial strains** | **Description** | **Source/reference** |
|---|---|---|
| *S. dublin* | Wild-type isolated from cheese | NCTC |
| CVD908 | Typhi Ty2 *ΔaroC ΔaroD* | Hone (1991) Vaccine 9, 810 |
| BRD948 | Typhi Ty2 *ΔaroC ΔaroD ΔhtrA* | Tacket (1997) Inf & Immun 65,452 |
| BRD1116 | Typhi Ty2 *ΔaroA ΔaroD ΔhtrA* | Lowe (1999) Inf & Immun 67, 700 |
| XL10-Gold | Transformation proficient laboratory strain. | Stratagene |
| | | |

| **Plasmids** | **Description** | **Source/reference** |
|---|---|---|
| pPCR-Script | Vector for cloning *Pfu* derived PCR fragments. | Stratagene |
| pHUR3 | Plasmid expressing ureAB from *PhtrA,* ampR, pBR322 origin of replication. | |
| pHUR3*rsd* | Derivative of pHUR3 incorporating *rsd-crs* cassette derived from *S. dublin* virulence plasmid. | |
| pHUR3*rsd* Δ⁵⁹⁷⁴ | Derivative of pHUR3 incorporating *rsd crs* loci, intervening sequence deleted. | |
| pH3RC1 | Derivative of pHLTR3*rsd*Δ⁵⁹⁷⁴ in which *EcoRI* site proximal to *crs* is deleted. | |
| pH3RC2 | Derivative of pH3RC1 which included the *rrnB* 5S transcriptional terminator immediately upstream of *PhtrA.* | |
| pH3RC3 | Derivative of pH3RC2 in which amp^{R} is replaced with kan^{R.} | |

**Table 2. Oligonucleotides**

| Name | Nucleotide sequence | Target and comments |
|---|---|---|
| 5959 | 5'TCATTTTATAAC GCGAAGTTCCGG3' | *ureA.* Used to confirm linkage and orientation of other plasmid components. |
| 5960 | 5'GGCGAATTCCCG GGTAAGTGTGGAT ATGTG3' | *crs.* Includes 5'-overhang with *EcoRI* site. |
| 5961 | 5'GGCGAATTCCGG ATGGCCTGTTGCA GGC3' | *rsd.* Includes 5'-overhang with *EcoRI.* site. |
| 5962 | 5'CTCGTGCATTTTA CTGATGC3' | *crs* |
| 5974 | 5'GCATCAGTAAA.A TGCACGAGCCTTT ATACAGGGTTCAG AC3' | *rsd* and fusion to *crs.* Bases 1 to 20 complementary to 5962. |
| 5989 | 5'TCATCATGAACA ATAAAACTGTCTG C3' | Kanamycin GenBlock. Includes 5'-overhang with *Bsp*HI site. |
| 5990 | 5'GCCTCATGATCT GATCCTTCAACTCA GC3' | Kanamycin GenBlock. Includes 5'-overhang with *Bsp*HI site. |
| 5991 | 5'GCCGAATTCACC GTATCTGTGGGGG GATG3' | *rrnB* 5S transcriptional terminator. Includes 5'-overhang with *Eco*RI site. |
| 5992 | 5'GCGAAATTTCCC CATGCGAGAGTAG GG3' | *rrnB* 5S transcriptional terminator. Includes 5'-overhang with *Apo*I site. |

## Claims

1. A vaccine comprising a pharmaceutically acceptable carrier or diluent and a bacterium, wherein the bacterium contains:
(i) a DNA sequence encoding a site-specific recombinase; and
(ii) a plasmid comprising a recognition element for the recombinase and a DNA sequence encoding a heterologous polypeptide.

2. A vaccine according to claim 1, wherein the heterologous polypeptide is an antigen of a pathogen.

3. A vaccine according to claim 1 or 2, wherein the plasmid contains the DNA sequence encoding the site-specific recombinase.

4. A vaccine according to any one of the preceding claims, wherein the site-specific recombinase is a member of the resolvase family of site-specific recombinases.

5. A vaccine according to claim 4, wherein the site-specific recombinase is the Rsd resolvase and the recognition element is the *crs* recognition element.

6. A vaccine according to claim 5 wherein the Rsd resolvase and the *crs* recognition element are less than 0.5kb apart on the plasmid.

7. A vaccine according to claim 5 or 6 wherein the Rsd resolvase is encoded by
(a) a DNA molecule comprising the nucleotide sequence from positions 680 to 1459 of SEQ ID NO: 1,
(b) a DNA molecule which hybridises to the complement of (a), or
(c) a DNA molecule which encodes the same amino acid sequence as the DNA molecule of (a) or (b) but which is a degenerate form of the DNA molecule of
(a) or (b).

8. A vaccine according to claim 5, 6 or 7 wherein the *crs* recognition element has the sequence of
(a) nucleotides 54 to 556 of SEQ ID NO: 1 or the complement thereof, or
(b) a DNA molecule which hybridises to a DNA molecule comprising the sequence of nucleotides 53 to 556 of SEQ ID NO: 1 or to the complement thereof.

9. A vaccine according to any one of the preceding claims, wherein there is a transcriptional termination sequence between the recognition element and the promoter of the gene encoding the heterologous polypeptide.

10. A vaccine according to any one of the preceding claims, wherein expression of the polypeptide is driven by the *nirB, pag C*, *ssaH* or *htrA* promoter.

11. A vaccine according to any one of the preceding claims, wherein the bacterium is from the genus *Escherichia, Salmonella, Shigella, Vibrio, Neisseria, Bordetella, Listeria or Mycobacterium.*

12. A vaccine according to claim 11, wherein the bacterium is *Escherichia coli, Salmonella typhimurium, Salmonella typhi, Salmonella enteritidis, Salmonell*a *choleraesuis, Salmonella dublin, Neisseria gonorrhoeae, Neisseria menigitidis, Shigella flexneri, Shigelia dysenteriae, Shigella sonnei, Vibrio Cholerae* or *Mycobacterium tuberculosis.*

13. A vaccine according to any one of the preceding claims, wherein the bacterium is an attenuated strain of a pathogenic bacterium.

14. A vaccine according to claim 13, wherein the bacterium is an attenuated strain of *Salmonella typhi.*

15. A vaccine according to claim 13, wherein the bacterium is an attenuated strain of enterotoxigenic *E. coli* (ETEC), enteropathogenic *E. coli* (EPEC), enteroinvasive *E.coli* (EIEC) or enterohemorrhagic *E.coli* (EHEC).

16. A vaccine according to claim 13, wherein the bacterium is *Shigella flexneri* or *Shigella sonnei.*

17. A vaccine according to any one of claims 13 to 16, wherein the bacterium is attenuated by a non-reverting mutation in at least one of the following genes: an *aro* gene, *a pur* gene, *ompC, ompF, ompR, htrA, galE, cya, crp,* and *phoP.*

18. A vaccine according to any one of claims 13 to 17 wherein the bacterium is attenuated by a non-reverting mutation in one, two, three or four separate genes.

19. A vaccine according to any one of claims 13 to 18, wherein the bacterium is attenuated by (i) a non-reverting mutation in the *htrA* gene or at least one *omp* gene and (ii) a non-reverting mutation in at least one *aro* gene.

20. A vaccine according to any one of claims 13 to 18, wherein the bacterium is attenuated by a non-reverting mutation in each of the *aroC* gene, the *ompF* gene and the *ompC* gene.

21. A vaccine according to any one of claims 13 to 19, wherein the bacterium is attenuated by a non-reverting mutation in each of two discrete *aro* genes.

22. A vaccine according to claim 21 wherein the *aro* genes are *aroA* and *aroC, aroA* and *aroD* or *aroC* and *aroD.*

23. A vaccine according to any one of claims 1 to 9 and 1 to 22, wherein expression of the heterologous polypeptide is driven by a eukaryotic expression cassette.

24. A vaccine according to claim 23, wherein the bacterium is an invasive bacterium.

25. A vaccine according to any one of claims 2 to 24 wherein the heterologous antigen is a *Helicobacter pylori* antigen.

26. A bacterium as defined in any one of the preceding claims, wherein the heterologous polypeptide is an antigen of a pathogen.

27. A plasmid as defined in any one of claims 1 to 10, 23 and 25, wherein the heterologous polypeptide is an antigen of a pathogen.

28. A vaccine according to any one of claims 1 to 25 or a bacterium according to claim 26 for use in a method of vaccinating a human or animal.

29. Use of a bacterium according to claim 26 for the manufacture of a medicament for vaccinating a human or animal.

## Patentansprüche

1. Impfstoff, umfassend einen pharmazeutisch akzeptablen Träger oder Verdünnungsmittel und ein Bakterium, wobei das Bakterium enthält:
(i) eine DNA-Sequenz, die für eine ortsspezifische Rekombinase codiert; und
(ii) ein Plasmid, umfassend ein Erkennungselement für die Rekombinase und eine DNA-Sequenz, die für ein heterologes Polypeptid codiert.

2. Impfstoff nach Anspruch 1, worin das heterologe Polypeptid ein Antigen eines Pathogens ist.

3. Impfstoff nach Anspruch 1 oder 2, worin das Plasmid die DNA-Sequenz enthält, die für die ortsspezifische Rekombinase codiert.

4. Impfstoff nach einem der vorangehenden Ansprüche, worin die ortsspezifische Rekombinase ein Mitglied der Resolvase-Familie der ortsspezifischen Rekombinasen ist.

5. Impfstoff nach Anspruch 4, worin die ortsspezifische Rekombinase die Rsd-Resolvase ist und das Erkennungselement das crs-Erkennungselement ist.

6. Impfstoff nach Anspruch 5, worin die Rsd-Resolvase und das crs-Erkennungselement weniger als 0,5 kb auf dem Plasmid voneinander entfernt sind.

7. Impfstoff nach Anspruch 5 oder 6, worin die Rsd-Resolvase codiert ist durch
(a) ein DNA-Molekül, umfassend die Nukleotidsequenz von Positionen 680 bis 1459 der SEQ ID Nr. 1,
(b) ein DNA-Molekül, welches an das Komplement von (a) hybridisiert, oder
(c) ein DNA-Molekül, welches für dieselbe Aminosäuresequenz wie das DNA-Molekül von (a) oder (b) codiert, doch welches eine degenerierte Form des DNA-Moleküls von (a) oder (b) ist.

8. Impfstoff nach Anspruch 5, 6 oder 7, worin das crs-Erkennungselement die Sequenz aufweist von
(a) Nukleotiden 54 bis 556 der SEQ ID Nr. 1 oder des Komplements davon, oder
(b) einem DNA-Molekül, welches an ein DNA-Molekül, umfassend die Sequenz des Nukleotide 53 bis 556 der SEQ ID Nr. 1 oder des Komplements davon, hybridisiert.

9. Impfstoff nach einem der vorangehenden Ansprüche, worin sich eine Transkriptionsterminationssequenz zwischen dem Erkennungselement und dem Promotor des Gens, das für das heterologe Polypeptid codiert, befindet.

10. Impfstoff nach einem der vorangehenden Ansprüche, worin die Expression des Polypeptids durch den *nirB-, pag C-, ssaH-* oder *htrA*-Promotor gesteuert wird.

11. Impfstoff nach einem der vorangehenden Ansprüche, worin das Bakterium von der Gattung *Escherichia, Salmonella, Shigella, Vibrio, Neisseria, Bordetella, Listeria* oder *Mycobacterium* ist.

12. Impfstoff nach Anspruch 11, worin das Bakterium *Escherichia coli, Salmonella typhimurium, Salmonella typhi, Salmonella enteritidis, Salmonella choleraesuis, Salmonella dublin, Neisseria gonorrhoeae, Neisseria minigitidis, Shigella flexneri, Shigella dysenteriae, Shigella sonnei, Vibrio cholerae* oder *Mycobacterium tuberculosis* ist.

13. Impfstoff nach einem der vorangehenden Ansprüche, worin das Bakterium ein abgeschwächter Stamm eines pathogenen Bakteriums ist.

14. Impfstoff nach Anspruch 13, worin das Bakterium ein abgeschwächter Stamm von *Salmonella typhi* ist.

15. Impfstoff nach Anspruch 13, worin das Bakterium ein abgeschwächter Stamm von enterotoxigenem *E.coli* (ETEC), enteropathogenem *E.coli* (EPEC), enteroinvasivem *E.coli* (EIEC) oder enterohämorrhagischem *E.coli* (EHEC) ist.

16. Impfstoff nach Anspruch 13, worin das Bakterium *Shigella flexneri* oder *Shigella sonnei* ist.

17. Impfstoff nach einem der Ansprüche 13 bis 16, worin das Bakterium durch eine nicht-revertiende Mutation in wenigstens einem der folgenden Gene abgeschwächt ist: einem *aro*-Gen, einem *pur*-Gen, *ompC, ompF, ompR, htrA, GaIE, cya, crp* und *phoP.*

18. Impfstoff nach einem der Ansprüche 13 bis 17, worin das Bakterium durch eine nicht-revertierende Mutation in ein, zwei, drei oder vier separaten Genen abgeschwächt ist.

19. Impfstoff nach einem der Ansprüche 13 bis 18, worin das Bakterium durch (i) eine nicht-revertierende Mutation in dem *htrA*-Gen oder wenigstens einem *omp-*Gen und (ii) eine nicht-revertierende Mutation in wenigstens einem *aro*-Gen abgeschwächt ist.

20. Impfstoff nach einem der Ansprüche 13 bis 18, worin das Bakterium durch eine nicht-revertierende Mutation in jedem von dem *aroc-Gen,* dem *ompF*-Gen und dem *ompC-*Gen abgeschwächt ist.

21. Impfstoff nach einem der Ansprüche 13 bis 19, worin das Bakterium durch eine nicht-revertierende Mutation in jedem von zwei unterschiedlichen *aro*-Genen abgeschwächt ist.

22. Impfstoff nach Anspruch 21, worin die *aro-Gene aroA* und *aroC, aroA* und *aroD* oder *aroC* und *aroD* sind.

23. Impfstoff nach einem der Ansprüche 1 bis 9 und 11 bis 22, worin die Expression des heterologen Polypeptids durch eine eukaryotische Expressionskassette gesteuert wird.

24. Impfstoff nach Anspruch 23, worin das Bakterium ein invasives Bakterium ist.

25. Impfstoff nach einem der Ansprüche 2 bis 24, worin das heterologe Antigen ein *Helicobacter pylori*-Antigen ist.

26. Bakterium, wie in einem der vorangehenden Ansprüche definiert, worin das heterologe Polypeptid ein Antigen eines Pathogens ist.

27. Plasmid, wie in einem der Ansprüche 1 bis 10, 23 und 25 definiert, worin das heterologe Polypeptid ein Antigen eines Pathogens ist.

28. Impfstoff nach einem der Ansprüche 1 bis 25 oder ein Bakterium nach Anspruch 26 zur Verwendung bei einer Methode zum Impfen eines Menschen oder Tiers.

29. Verwendung eines Bakteriums nach Anspruch 26 für die Herstellung eines Medikaments zum Impfen eines Menschen oder Tiers.

## Revendications

1. Vaccin comprenant un véhicule ou diluant pharmaceutiquement acceptable et une bactérie, dans lequel la bactérie contient :
(i) une séquence d'ADN codant pour une recombinase site spécifique ; et
(ii) un plasmide comprenant un élément de reconnaissance pour ladite recombinase et une séquence d'ADN codant pour un polypeptide hétérologue.

2. Vaccin selon la revendication 1, dans lequel le polypeptide hétérologue est un antigène d'un pathogène.

3. Vaccin selon la revendication 1 ou 2, dans lequel le plasmide contient la séquence d'ADN codant pour la recombinase site spécifique.

4. Vaccin selon l'une quelconque des revendications précédentes, dans lequel la recombinase site spécifique est un membre de la famille des résolvases aux sein des recombinases site spécifiques.

5. Vaccin selon la revendication 4, dans lequel la recombinase site spécifique est la résolvase Rsd et l'élément de reconnaissance est l'élément de reconnaissance *crs*.

6. Vaccin selon la revendication 5 dans lequel la résolvase Rsd et l'élément de reconnaissance crs sont distants de moins de 0,5 kb sur le plasmide.

7. Vaccin selon la revendication 5 ou 6 dans lequel la résolvase Rsd est codée par
(a) une molécule d'ADN comprenant la séquence nucléotidique des positions 680 à 1459 de SEQ ID nO :1,
(b) une molécule d'ADN qui s'hybride avec le complément de (a), ou
(c) une molécule d'ADN qui code pour la même séquence d'acides aminés que la molécule d'ADN de (a) ou (b) mais qui est une forme dégénérée de la molécule d'ADN de (a) ou (b).

8. Vaccin selon la revendication 5, 6 ou 7 dans lequel l'élément de reconnaissance *crs* a la séquence
(a) des nucléotides 54 à 556 de SEQ ID NO :1 ou son complément, ou
(b) d'une molécule d'ADN qui s'hybride avec une molécule d'ADN comprenant la séquence des nucléotides 53 à 556 de SEQ ID NO :1 ou son complément.

9. Vaccin selon l'une quelconque des revendications précédentes, dans lequel il y a une séquence de terminaison transcriptionnelle entre l'élément de reconnaissance et le promoteur du gène codant pour le polypeptide hétérologue.

10. Vaccin selon l'une quelconque des revendications précédentes, dans lequel l'expression du polypeptide est commandée par le promoteur *nirB, pagC, ssaH* ou *htrA*.

11. Vaccin selon l'une quelconque des revendications précédentes, dans lequel la bactérie est du genre *Escherichia, Salmonella, Shigella, Vibrio, Neisseria, Bordetella, Listeria* ou *Mycobacterium.*

12. Vaccin selon la revendication 11, dans lequel la bactérie est *Escherichia coli, Salmonella typhimurium, Salmonella typhi, Salmonella enteritidis, Salmonella choleraesuis, Salmonella dublin, Neisseria gonorrhoeae, Neisseria meningitidis, Shigella flexneri, Shigella dysenteriae, Shigella sonnei, Vibrio cholerae* ou *Mycobacterium tuberculosis.*

13. Vaccin selon l'une quelconque des revendications précédentes, dans lequel la bactérie est une souche atténuée d'une bactérie pathogène.

14. Vaccin selon la revendication 13, dans lequel la bactérie est une souche atténuée de *Salmonella typhi.*

15. Vaccin selon la revendication 13, dans lequel la bactérie est une souche atténuée de *E. coli* entérotoxinogène (ETEC), de *E. coli* entéropathogène (EPEC), *E. coli* entéroinvasive (EIEC) ou *E. coli* entérohémorragique (EHEC).

16. Vaccin selon la revendication 13, dans lequel la bactérie est *Shigella flexneri* ou *Shigella sonnei.*

17. Vaccin selon l'une quelconque des revendications 13 à 16, dans lequel la bactérie est atténuée par une mutation non réversible dans au moins un des gènes suivants : un gène *aro,* un gène *pur, ompC, ompF, ompR, htrA, galE, cya, crp,* et *phoP.*

18. Vaccin selon l'une quelconque des revendications 13 à 17 dans lequel la bactérie est atténuée par une mutation non réversible dans un, deux, trois ou quatre gènes séparés.

19. Vaccin selon l'une quelconque des revendications 13 à 18, dans lequel la bactérie est atténuée par (i) une mutation non réversible dans le gène *htrA* ou au moins un gène *omp* et (ii) une mutation non réversible dans au moins un gène *aro*.

20. Vaccin selon l'une quelconque des revendications 13 à 18, dans lequel la bactérie est atténuée par une mutation non réversible dans chacun des gènes *aroC, ompF* et *ompC.*

21. Vaccin selon l'une quelconque des revendications 13 à 19, dans lequel la bactérie est atténuée par une mutation non réversible dans chacun des deux gènes *aro* séparés.

22. Vaccin selon la revendication 21 dans lequel les gènes *aro* sont *aroA* et *aroC, aroA* et *aroD* ou *aroC* et *aroD.*

23. Vaccin selon l'une quelconque des revendications 1 à 9 et 11 à 22, dans lequel l'expression du polypeptide hétérologue est commandée par une cassette d'expression eucaryote.

24. Vaccin selon la revendication 23, dans lequel la bactérie est une bactérie invasive.

25. Vaccin selon l'une quelconque des revendications 2 à 24 dans lequel l'antigène hétérologue est un antigène *d'Helicobacter pylori.*

26. Bactérie telle que définie dans l'une quelconque des revendications précédentes, dans laquelle le polypeptide hétérologue est un antigène d'un pathogène.

27. Plasmide tel que défini dans l'une quelconque des revendications 1 à 10, 23 et 25, dans lequel le polypeptide hétérologue est un antigène d'un pathogène.

28. Vaccin selon l'une quelconque des revendications 1 à 25 ou bactérie selon la revendication 26 utilisable dans une méthode de vaccination d'un humain ou d'un animal.

29. Utilisation d'une bactérie selon la revendication 26 pour la préparation d'un médicament pour vacciner un humain ou un animal.


### SEQUENCE LISTING

<110> ACAMBIS RESEARCH LIMITED
<120> STABILISATION OF PLASMID INHERITANCE IN BACTERIA
<130> N79943A
<140>
   <141>
<160> 11
<170> PatentIn Ver. 2.1
<210> 1
   <211> 1811
   <212> DNA
   <213> Artificial Sequence
<220>
   <223> Description of Artificial Sequence: plasmid
<220>
   <221> CDS
   <222> (680)..(1459)
   <223> Rsd coding sequence
<220>
   <221> misc_feature
   <222> (54)..(556)
   <223> crs recognition element
<220>
   <221> misc_feature
   <222> (1549)..(1593)
   <223> rrnB T1
<220>
   <221> misc_feature
   <222> (1718)..(1753)
   <223> rrnB T2
<220>
   <221> primer-bind
   <222> (6)..(31)
   <223> Primer 5960
<220>
   <221> primer_bind
   <222> (572)..(591)
   <223> Primer 5962
<220>
   <221> primer_bind
   <222> (592)..(611)
   <223> Primer 5974
<220>
   <221> primer_bind
   <222> (1490)..(1513)
   <223> Primer 5961
<220>
   <221> primer_bind
   <222> (1510)..(1532)
   <223> Primer 5992
<220>
   <221> primer_bind
   <222> (1630)..(1654)
   <223> Primer 5983
<220>
   <221> primer_bind
   <222> (1792)..(1811)
   <223> Primer 5991
<400> 1
   gaattaattc ccgggtaagt gtggatatgt gatgcatatc cggaatgatg ttcattgcac 60
   cgggtgtgtg catatgttca gtataggcat atgttcggca tatgtataga ttcagcatat 120
   gcagatggtt aggatatgcg tatgccacgg atatgcagat gtcaggcata tggatatgtt 180
   cagcctatgt gattttctct ctttccccgc gcagatccga tttctggtgg tcccggatct 240
   tctttatcct caccggtgcg ccgcgacccg gtaacacaag atgtagcggc tcagtcatct 300
   gctgttgcta ccagatgttg tgttataccc cttccctgtg acccggcgtg cagggcagaa 360
   aagaccgctt tgcttattcc ctgaccgtgg tacacttccg gaaagtcgtg aatagacaaa 420
   aaatcggatc acctgaaaat agccgtcctg taattccgtt acgtttttat aagttggatt 480
   gtaattgcat gtggttaaag tttcattatg aaaaagatga gtgatttact aatagcgtga 540
   taacagtatg cgttttaagg attgaaaaca tgcatcagta aaatgcacga gcctttatac 600
   agggttcaga ctgaaaccag agatgactcc ggggaaaaaa catgatgtcc cggaatattt 660
   cacttaacgg agagaggac atg agt cag cca ccg tta cct gct gtc tgt acg  712
                       Met Ser Gln Pro Pro Leu Pro Ala Val Cys Thr
                        1               5                   10
   cag gca gcg tcc gcc ctg ctg ccg gtg gcc att gat tac ccg gcg gcc  760
   Gln Ala Ala Ser Ala Leu Leu Pro Val Ala Ile Asp Tyr Pro Ala Ala
                15                  20                  25
   ctc gca ctg cgc cag atg gca atg cag cat gac gac tac ccg aaa tat  808
   Leu Ala Leu Arg Gln Met Ala Met Gln His Asp Asp Tyr Pro Lys Tyr
            30                  35                  40
   ctg ctg gct ccg gag gta agt gcc ctg ctc cac tac gtc cct gat ctg  856
   Leu Leu Ala Pro Glu Val Ser Ala Leu Leu His Tyr Val Pro Asp Leu
        45                  50                  55
   cat cgc agg atg ctg ctg gcc act cta tgg aat acc ggc gca cgg att  904
   His Arg Arg Met Leu Leu Ala Thr Leu Trp Asn Thr Gly Ala Arg Ile
    60                  65                  70                  75
   aac gag gcg ctg gcc ttg aca cgg gga gac ttt tcg ctg gca cca cct  952
   Asn Glu Ala Leu Ala Leu Thr Arg Gly Asp Phe Ser Leu Ala Pro Pro
                    80                  85                  90
   tat ccg ttt gtg cag ctg gcc act ctg aaa cag cgg gcg gaa aag gcg  1000
   Tyr Pro Phe Val Gln Leu Ala Thr Leu Lys Gln Arg Ala Glu Lys Ala
                95                 100                 105
   gcc aga acg gca gga cgg atg ccg tcc ggc agc cag ccc cac cgc ttg  1048
   Ala Arg Thr Ala Gly Arg Met Pro Ser Gly Ser Gln Pro His Arg Leu
           110                 115                 120
   gtg ccg ctt tct gat aac cag tat gtc agc gaa ctg cag atg atg gtg  1096
   Val Pro Leu Ser Asp Asn Gln Tyr Val Ser Glu Leu Gln Met Met Val
       125                 130                 135
   gcc acg ctg aaa atc ccg ctg gag cgt cgt aac aga cgt acc ggc aga   1144
   Ala Thr Leu Lys Ile Pro Leu Glu Arg Arg Asn Arg Arg Thr Gly Arg
   140                 145                 150                 155
   acg gaa aag gcg cgc ctc tgg gag atc acc gac cgg acg gtc cgt acc   1192
   Thr Glu Lys Ala Arg Leu Trp Glu Ile Thr Asp Arg Thr Val Arg Thr
                   160                 165                 170
   tgg att ggg gag gcg gtt gag gcc gca gcg gct gac gac gtg acg ttc   1240
   Trp Ile Gly Glu Ala Val Glu Ala Ala Ala Ala Asp Asp Val Thr Phe
               175                 180                 185
   tca gtc ccg gtg aca ccc cat aca ttc cgc cac tcc tat gcg atg cac   1288
   Ser Val Pro Val Thr Pro His Thr Phe Arg His Ser Tyr Ala Met His
           190                 195                 200
   atg ctg tac gcc ggc ata ccg ctg aag gtg ctg cag gcg ctg atg gga   1336
   Met Leu Tyr Ala Gly Ile Pro Leu Lys Val Leu Gln Ala Leu Met Gly
       205                 210                 215
   cac aaa tcg gtg agc tcg acg gaa gtg tac acg aaa gtg ttt gcg ctt   1384
   His Lys Ser Val Ser Ser Thr Glu Val Tyr Thr Lys Val Phe Ala Leu
   220                 225                 230                 235
   gat gtt gcc gca cga cac cgg gtg cag ttt cag atg ccg ggt gct gat   1432
   Asp Val Ala Ala Arg His Arg Val Gln Phe Gln Met Pro Gly Ala Asp
                   240                 245                 250
   gca gtg gct atg ctc aaa gga ggt tca tagagacgtg tatgcatttt         1479
   Ala Val Ala Met Leu Lys Gly Gly Ser
               255                 260
   tcagcctttc gcctgcaaca ggccatccgg aatttcccca tgcgagagta gggaactgcc 1539
   aggcatcaaa taaaacgaaa ggctcagtcg aaagactggg cctttcgttt tatctgttgt 1599
   ttgtcggtga acgctctcct gagtaggaca aatccgccgg gagcggattt gaacgttgcg 1659
   aagcaacggc ccggagggtg gcgggcagga cgcccgccat aaactgccag gcatcaaatt 1719
   aagcagaagg ccatcctgac ggatggcctt tttgcgtttc tacaaactct tcctgtcgtc 1779
   atatctacaa gccatccccc cacagatacg gt                               1811
<210> 2
   <211> 260
   <212> PRT
   <213> Artificial Sequence.
   <223> Description of Artificial Sequence: plasmid
<400> 2
   Met Ser Gln Pro Pro Leu Pro Ala Val Cys Thr Gln Ala Ala Ser Ala
    1               5                  10                  15
   Leu Leu Pro Val Ala Ile Asp Tyr Pro Ala Ala Leu Ala Leu Arg Gln
               20                  25                  30
   Met Ala Met Gln His Asp Asp Tyr Pro Lys Tyr Leu Leu Ala Pro Glu
           35                  40                  45
   Val Ser Ala Leu Leu His Tyr Val Pro Asp Leu His Arg Arg Met Leu
       50                  55                  60
   Leu Ala Thr Leu Trp Asn Thr Gly Ala Arg Ile Asn Glu Ala Leu Ala
   65                  70                  75                  80
   Leu Thr Arg Gly Asp Phe Ser Leu Ala Pro Pro Tyr Pro Phe Val Gln
                   85                  90                  95
   Leu Ala Thr Leu Lys Gln Arg Ala Glu Lys Ala Ala Arg Thr Ala Gly
               100                 105                 110
   Arg Met Pro Ser Gly Ser Gln Pro His Arg Leu Val Pro Leu Ser Asp
           115                 120                 125
   Asn Gln Tyr Val Ser Glu Leu Gln Met Met Val Ala Thr Leu Lys Ile
       130                 135                 140
   Pro Leu Glu Arg Arg Asn Arg Arg Thr Gly Arg Thr Glu Lys Ala Arg
   145                 150                 155                 160
   Leu Trp Glu Ile Thr Asp Arg Thr Val Arg Thr Trp Ile Gly Glu Ala
                   165                 170                 175
   Val Glu Ala Ala Ala Ala Asp Asp Val Thr Phe Ser Val Pro Val Thr
               180                 185                 190
   Pro His Thr Phe Arg His Ser Tyr Ala Met His Met Leu Tyr Ala Gly
           195                 200                 205
   Ile Pro Leu Lys Val Leu Gln Ala Leu Met Gly His Lys Ser Val Ser
   210 215 220
   Ser Thr Glu Val Tyr Thr Lys Val Phe Ala Leu Asp Val Ala Ala Arg
   225 230 235 240
   His Arg Val Gln Phe Gln Met Pro Gly Ala Asp Ala Val Ala Met Leu
   245 250 255
   Lys Gly Gly Ser
   260
<210> 3
   <211> 24
   <212> DNA
   <213> Artificial Sequence
<220>
   <223> Description of Artificial Sequence: PRIMER
<400> 3
   tcattttata acgcgaagtt ccgg 24
<210> 4
   <211> 30
   <212> DNA
   <213> Artificial Sequence
<220>
   <223> Description of Artificial Sequence: PRIMER
<400> 4
   ggcgaattcc cgggtaagtg tggatatgtg 30
<210> 5
   <211> 28
   <212> DNA
   <213> Artificial Sequence
<220>
   <223> Description of Artificial Sequence: PRIMER
<400> 5
   ggcgaattcc ggatggcctg ttgcaggc 28
<210> 6
   <211> 20
   <212> DNA
   <213> Artificial Sequence
<220>
   <223> Description of Artificial Sequence: PRIMER
<400> 6
   ctcgtgcatt ttactgatgc 20
<210> 7
   <211> 40
   <212> DNA
   <213> Artificial Sequence
<220>
   <223> Description of Artificial Sequence: PRIMER
<400> 7
   gcatcagtaa aatgcacgag cctttataca gggttcagac 40
<210> 8
   <211> 26
   <212> DNA
   <213> Artificial Sequence
<220>
   <223> Description of Artificial Sequence: PRIMER
<400> 8
   tcatcatgaa caataaaact gtctgc 26
<210> 9
   <211> 28
   <212> DNA
   <213> Artificial Sequence.
<220>
   <223> Description of Artificial Sequence: PRIMER
<400> 9
   gcctcatgat ctgatccttc aactcagc 28
<210> 10
   <211> 29
   <212> DNA
   <213> Artificial Sequence
<220>
   <223> Description of Artificial Sequence: PRIMER
<400> 10
   gccgaattca ccgtatctgt ggggggatg 29
<210> 11
   <211>. 27
   <212> DNA
   <213> Artificial Sequence
<220>
   <223> Description of Artificial-Sequence: PRIMER
<400> 11
   gcgaaatttc cccatgcgag agtaggg 27